# EUROPEAN PATENT APPLICATION

(11) **EP 4 716 378 A1**
(43) Date of publication of application: **25.03.2026**
(21) Application number: 25203727.0
(22) Date of filing: 22.09.2025
(51) Int. Cl.: H05G 1/12, H05G 1/32

(54) **METHODS OF CONTROLLING BIPOLAR HIGH-VOLTAGE GENERATORS AND X-RAY SYSTEMS**

(30) Priority: 20.09.2024 CN 202411321292
(71) Applicant: Wuhan United Imaging Healthcare Co., Ltd., WuHan, Hubei 430206 (CN)
(72) Inventor: DUAN,, Yaoqiang, Wuhan,, 430206 (CN); ZHANG,, Tieshan, Wuhan, 430206 (CN); CAO,, Bin, Wuhan, 430206 (CN); CHU,, Xu, Wuhan, 430206 (CN)
(74) Representative: Wang, Bo

(57) **Abstract**

Disclosed herein are a method of controlling a bipolar high-voltage generator and an X-ray system. The bipolar high-voltage generator is connected to a load for supplying a cathode voltage and an anode voltage to the load. The bipolar high-voltage generator includes a first transformer and a second transformer. The first transformer is provided in an output circuit of the anode voltage, and the second transformer is provided in an output circuit of the cathode voltage. The method of controlling includes: obtaining a target voltage difference of the bipolar high-voltage generator, wherein the target voltage difference is within a preset range; and adjusting a working state of at least one of the first transformer and the second transformer based on the target voltage difference. The target voltage difference is defined as a difference between an absolute value of a target anode voltage and an absolute value of a target cathode voltage output from the bipolar high-voltage generator.

## Description

### CROSS-REFERENCE

This application claims priority to Chinese Patent Application No. 202411321292.9, filed on September 20, 2024, the entire contents of which are incorporated herein by reference.

### TECHNICAL FIELD

The present disclosure relates to the field of high-voltage generators, and in particular, to a method of controlling a bipolar high-voltage generator and an X-ray system.

### BACKGROUND

In Computed Tomography (CT) systems, X-rays are produced mainly by loading a high-voltage (KV) on an X-ray tube. The current inside the X-ray tube is a current generated by the movement of free electrons emitted by a filament at a cathode end of the X-ray tube. As the free electrons reach an anode end of the X-ray tube, a portion of the free electrons are collected by a tube wall of the X-ray tube due to scattering. For a bipolar X-ray tube, the tube wall is grounded, and a portion of the free electrons emitted from the cathode end reaches the anode end to form an anode circuit current, while the remaining portion flows through the grounded tube wall to form a circuit current. Therefore, from the perspective of circuit principles, due to the presence of scattered electrons, a cathode current and an anode current of the bipolar X-ray tube are not equal. For the bipolar high-voltage generator, it operates under the condition of an unbalanced load between the cathode and the anode, resulting in an imbalance between a cathode voltage and an anode voltage output by the bipolar high-voltage generator.

Based on this, it is desirable to provide a method of controlling a bipolar high-voltage generator and an X-ray system, ensuring that the cathode voltage and the anode voltage output by the bipolar high-voltage generator are balanced.

### SUMMARY

One of the embodiments of the present disclosure provide a method of controlling a bipolar high-voltage generator, the bipolar high-voltage generator including a first transformer and a second transformer, the bipolar high-voltage generator being connected to a load for supplying a cathode voltage and an anode voltage to the load, the first transformer being provided in an output circuit of the anode voltage, and the second transformer being provided in an output circuit of the cathode voltage, the method comprising: obtaining a target voltage difference of the bipolar high-voltage generator, wherein the target voltage difference is within a preset range; and adjusting a working state of at least one of the first transformer and the second transformer based on the target voltage difference. The target voltage difference is defined as a difference between an absolute value of a target anode voltage and an absolute value of a target cathode voltage output from the bipolar high-voltage generator.

One of the embodiments of the present disclosure provide a method of controlling a bipolar high-voltage generator, the bipolar high-voltage generator including an inverter circuit, a first transformer, and a second transformer, the bipolar high-voltage generator being connected to a load, the bipolar high-voltage generator supplying a cathode voltage and an anode voltage to the load, the first transformer being provided in an output circuit of the anode voltage, and the second transformer being provided in an output circuit of the cathode voltage, the inverter circuit being coupled to an input of the first transformer and an input of the second transformer, the method comprising: obtaining a target voltage difference of the bipolar high-voltage generator, wherein the target voltage difference is within a preset range; and adjusting, by the inverter circuit, a working frequency of at least one of the first transformer and the second transformer based on the target voltage difference. The target voltage difference is defined as a difference between an absolute value of a target anode voltage and an absolute value of a target cathode voltage output from the bipolar high-voltage generator.

One of the embodiments of the present disclosure provide an X-ray system, comprising: an X-ray tube, the X-ray tube including a cathode and an anode; a bipolar high-voltage generator, the bipolar high-voltage generator being connected to the cathode and the anode of the X-ray tube, and the bipolar high-voltage generator supplying a cathode voltage to the cathode of the X-ray tube and an anode voltage to the anode of the X-ray tube, the bipolar high-voltage generator including an inverter circuit, a first transformer, and a second transformer, the first transformer being provided in an output circuit of the anode voltage, and the second transformer being provided in an output circuit of the cathode voltage, the inverter circuit being coupled to an input of the first transformer and an input of the second transformer. A difference between an absolute value of the anode voltage and an absolute value of the cathode voltage output from the bipolar high-voltage generator falls within a preset range.

### BRIEF DESCRIPTION OF THE DRAWINGS

The present disclosure will be further illustrated by way of exemplary embodiments, which will be described in detail by means of the accompanying drawings. These embodiments are not limiting, and in these embodiments, the same numbering denotes the same structure, wherein:
FIG. 1 is a schematic diagram illustrating a circuit of an X-ray system according to some embodiments of the present disclosure;
FIG. 2 is a schematic diagram illustrating a first structure of a transformer according to some embodiments of the present disclosure;
FIG. 3 is a schematic diagram illustrating a second structure of a transformer according to some embodiments of the present disclosure;
FIG. 4 is a schematic diagram illustrating a third structure of a transformer according to some embodiments of the present disclosure;
FIG. 5 is a diagram illustrating an exemplary module of a control system of a bipolar high-voltage generator according to some embodiments of the present disclosure.;
FIG. 6 is an exemplary flowchart illustrating a method of controlling a bipolar high-voltage generator according to some embodiments of the present disclosure;
FIG. 7 is a schematic diagram illustrating winding adjustment corresponding to a first structure of a transformer according to some embodiments of the present disclosure;
FIG. 8 is a schematic diagram illustrating winding adjustment corresponding to a first structure of a transformer according to some other embodiments of the present disclosure;
FIG. 9 is a schematic diagram illustrating winding adjustment corresponding to the first structure of the transformer according to some other embodiments of the present disclosure;
FIG. 10 is a schematic diagram illustrating winding adjustment corresponding to a third structure of a transformer according to some embodiments of the present disclosure;
FIG. 11 is a schematic diagram illustrating winding adjustment corresponding to a first structure of a transformer according to some embodiments of the present disclosure;
FIG. 12 is a schematic diagram illustrating winding adjustment corresponding to a third structure of a transformer according to some embodiments of the present disclosure;
FIG. 13 is an exemplary flowchart illustrating a process for determining an adjustment parameter according to some embodiments of the present disclosure;
FIG. 14 is an exemplary flowchart illustrating a method of controlling a bipolar high-voltage generator according to some other embodiments of the present disclosure;
FIG. 15 is a schematic diagram illustrating an equivalent circuit of a bipolar high-voltage generator according to some embodiments of the present disclosure; and
FIG. 16 is a graph illustrating an output voltage gain curve of a bipolar high-voltage generator according to some embodiments of the present disclosure.

### DETAILED DESCRIPTION

To more clearly illustrate the technical solutions of the embodiments of the present disclosure, the accompanying drawings required to be used in the description of the embodiments will be briefly described below. Obviously, the accompanying drawings in the following description are only some examples or embodiments of the present disclosure, and it is possible for a person of ordinary skill in the art to apply the present disclosure to other similar scenarios in accordance with these drawings without creative labor. Unless obviously obtained from the context or the context illustrates otherwise, the same numeral in the drawings refers to the same structure or operation.

It should be understood that, as used herein, the terms "system", "device", "unit" and/or "module" as used herein is a way to distinguish between different components, elements, parts, sections or assemblies at different levels. However, the words may be replaced by other expressions if other words accomplish the same purpose.

As shown in the present disclosure and claims herein, unless the context clearly suggests an exception, the words "a", "an ", "one", and/or "the" do not refer specifically to the singular, but may also include the plural. In general, the terms "including" and "comprising" only suggest the inclusion of explicitly identified steps and elements that do not constitute an exclusive list, and the method or device may also include other steps or elements.

Flowcharts are used in the present disclosure to illustrate operations performed by a system in accordance with embodiments of the present disclosure. It should be appreciated that the preceding or following operations are not necessarily performed in an exact sequence. Instead, steps may be processed in reverse order or simultaneously. Also, it is possible to add other operations to these processes or remove a step or steps from them.

In Computed Tomography (CT) systems, X-rays are produced mainly by loading a high-voltage (KV) on an X-ray tube. Depending on the structure of the X-ray tube, it may be categorized into unipolar and bipolar X-ray tubes, and the corresponding high-voltage generators are unipolar and bipolar high-voltage generators, respectively. The KV output by the unipolar high-voltage generator is generally a negative high-voltage to ground, and the KV output by the bipolar high-voltage generator has both positive and negative high-voltages.

The bipolar high-voltage generator may be viewed as two power circuits to ground at a load side, including an anode current (mA) circuit corresponding to the positive high-voltage and a cathode current circuit corresponding to the negative high-voltage. When the cathode current and the anode current are exactly equal, there is no current flowing through a midpoint branch connected to ground. In the practical application of an X-ray system, the current inside the X-ray tube is a current generated by the movement of free electrons emitted by the filament at the cathode end. As the electrons reach the anode end inside the X-ray tube, a portion of the electrons are emitted into a tube wall of the X-ray tube due to scattering. For the unipolar X-ray tube, the tube wall and the anode end are grounded together, and the scattered electrons and anode end electrons flow together in a grounded circuit. However, for the bipolar X-ray tube, the tube wall is grounded, and a portion of the free electrons emitted from the cathode reaches the anode end to form an anode circuit current, while the remaining portion flows through the grounded tube wall to form a circuit current. From the perspective of circuit principles, due to the presence of the scattered electrons, the cathode current and the anode current of the bipolar X-ray tube are not equal. Therefore, for the bipolar high-voltage generator, the cathode load and the anode load are unequal, i.e., the bipolar high-voltage generator operates under conditions of unbalanced cathode and anode load.

If the bipolar high-voltage generator operates under the conditions of unbalanced cathode and anode load, it may result in a phenomenon where the cathode voltage (also referred to as the cathode high-voltage) and the anode voltage (also referred to as the anode high-voltage) output from the bipolar high-voltage generator are unequal. Due to the electron scattering characteristics of the X-ray tube, the anode current is less than the cathode current, so that the anode high-voltage that is ultimately loaded to the anode of the X-ray tube may be greater than the cathode high-voltage loaded to the cathode.

One of the main difficulties in design of the bipolar high-voltage generator lies in insulation design of the high-voltage section. When the absolute value of the cathode high-voltage is equal to the absolute value of the anode high-voltage, insulation stresses on the cathode and the anode are minimized for the total voltage output. However, due to the aforementioned problem of imbalance (i.e., inequality) between the anode high-voltage and the cathode high-voltage, the anode output needs to meet a more demanding insulation design. The more demanding insulation design not only increases the design cost of the bipolar high-voltage generator, but also poses a greater challenge to the reliable operation of the bipolar high-voltage generator. Additionally, high-capacity X-ray tubes for the X-ray system generally use a rotating anode structure, so a portion of the anode of the X-ray tube is much more compact, which makes the insulation design of the high-voltage portion of the system more challenging. And, excessive anode high-voltage may also affect the safe operation of the X-ray tube.

In some embodiments, the anode high-voltage of the bipolar high-voltage generator may be reduced by a specific principle to realize the balance of the output voltage under the load imbalance condition of the bipolar high-voltage generator. An exemplary specific principle is: an additional inductor of a specific inductance is connected in series with the anode current circuit, thereby dividing the voltage by the series inductance during a power output phase. However, the additional series inductance disrupts the symmetry of the cathode current circuit and the anode current circuit of the bipolar high-voltage generator, imposing stricter requirements on the circuit design and the insulation design, while also increasing the product cost.

In some embodiments, by designing separate inverter circuits for the cathode current circuit and the anode current circuit on an inverter side of the high-voltage generator, the two inverter circuits independently control the cathode high voltage and the anode high voltage, respectively, to address the balance problem of the output voltage under the load imbalance condition in the bipolar high-voltage generator. However, the two sets of inverter circuits not only increase the cost of the bipolar high-voltage generator, but also increase the size and weight of the bipolar high-voltage generator. For the X-ray system operating on high-speed rotating gantries, a bulky and heavy bipolar high-voltage generator increases the overall operational load of the X-ray system and reduces its reliability.

To solve the above problem, embodiments of the present disclosure provide a method of controlling the bipolar high-voltage generator, including: obtaining a target voltage difference of the bipolar high-voltage generator, wherein the target voltage difference is within a preset range; and adjusting a working state of at least one of a first transformer and a second transformer of the bipolar high-voltage generator based on the target voltage difference. The target voltage difference is defined as a difference between an absolute value of a target anode voltage and an absolute value of a target cathode voltage output from the bipolar high-voltage generator. The working state may include at least one of a leakage inductance of the first transformer, a leakage inductance of the second transformer, a working frequency of the first transformer, or a working frequency of the second transformer. By adjusting the working state of at least one of the first transformer and the second transformer of the bipolar high-voltage generator, the difference between an absolute value of the anode voltage and an absolute value of the cathode voltage output from the bipolar high-voltage generator can be set to fall within the preset range, which achieves the balance of the output voltage of the bipolar high-voltage generator under the load imbalance condition without the need for additional structural components, ensuring the stability of the system without increasing insulation design requirements.

In some implementations, the bipolar high-voltage generator is connected to a load (e.g., the X-ray tube) for supplying the cathode voltage (also referred to as the cathode high-voltage) and the anode voltage (also referred to as the anode high-voltage) to the load. In some embodiments, the bipolar high-voltage generator includes the first transformer and the second transformer, the first transformer is provided in an output circuit of the anode voltage (i.e., the anode current circuit described above), and the second transformer is provided in an output circuit of the cathode voltage (i.e., the cathode current circuit described above). In some embodiments, a difference between a target leakage inductance of the first transformer and a target leakage inductance of the second transformer is a target leakage inductance difference, the target leakage inductance difference corresponds to the target voltage difference, and the target leakage inductance difference is not zero. In some embodiments, a working frequency of at least one of the first transformer and the second transformer is a target working frequency, and the target working frequency corresponds to the target voltage difference.

In some embodiments, the bipolar high-voltage generator includes an inverter circuit, the inverter circuit being coupled to an input of the first transformer and an input of the second transformer. In some embodiments, the bipolar high-voltage generator includes a resonant circuit, the inverter circuit being coupled, via the resonant circuit, to the input of the first transformer and the input of the second transformer. In some embodiments, the bipolar high-voltage generator includes a voltage multiplier rectifier circuit, an output of the first transformer and an output of the second transformer being connected to the inputs of the voltage multiplier rectifier circuit respectively; the voltage multiplier rectifier circuit outputs the anode high-voltage to an anode of the load and outputs the cathode high-voltage to a cathode of the load. In some embodiments, the bipolar high-voltage generator includes two voltage multiplier rectifier circuits, one of the two voltage multiplier rectifier circuits being connected to the output of the first transformer and the other being connected to the output of the second transformer; the two voltage multiplier rectifier circuits output the anode high-voltage to the anode of the load and the cathode high-voltage to the cathode of the load, respectively.

Embodiments of the present disclosure provide an X-ray system, comprising: an X-ray tube, the X-ray tube including a cathode and an anode; and a bipolar high-voltage generator, the bipolar high-voltage generator being connected to the cathode and the anode of the X-ray tube, and providing a cathode voltage to the cathode of the X-ray tube and an anode voltage to the anode of the X-ray tube. The bipolar high-voltage generator includes a first transformer and a second transformer, the first transformer being provided in an output circuit of the anode voltage, the second transformer being provided in an output circuit of the cathode voltage. A difference between an absolute value of the anode voltage and an absolute value of the cathode voltage output from the bipolar high-voltage generator falls within a preset range.

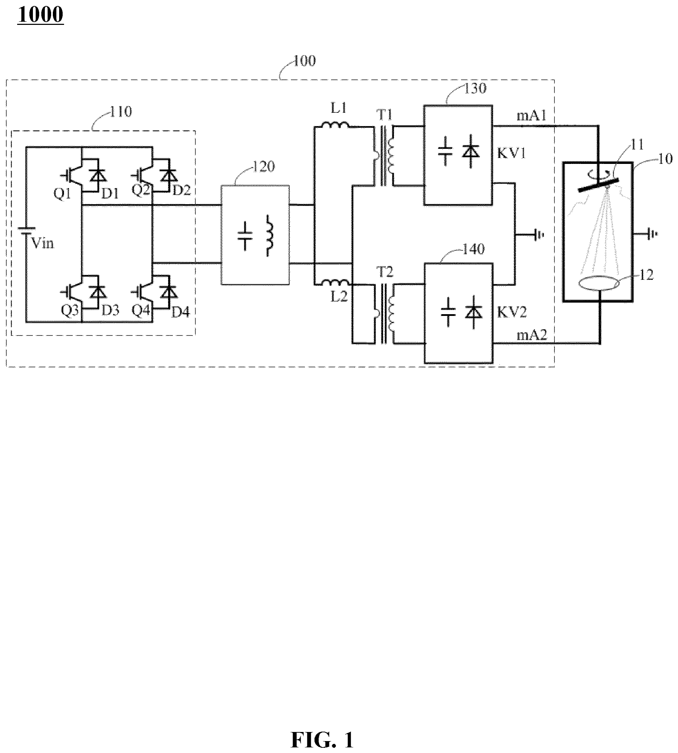

FIG. 1 is a schematic diagram illustrating a circuit of an X-ray system according to some embodiments of the present disclosure. As shown in FIG. 1, the X-ray system 1000 may include a bipolar high-voltage generator 100 and an X-ray tube 10.

The X-ray tube 10 may include an anode 11 and a cathode 12.

The bipolar high-voltage generator 100 is configured to connect the cathode 12 and the anode 11 of the X-ray tube 10, and supply a cathode high-voltage to the cathode 12 of the X-ray tube 10 and an anode high-voltage to the anode 11 of the X-ray tube 10. The cathode high-voltage and the anode high-voltage have opposite polarity. For example, the cathode high-voltage KV2 has a voltage range of -50 kV to -75 kV, and the anode high-voltage KV1 has a voltage range of +50 kV to +75 kV. In some embodiments, a difference between an absolute value of an anode voltage and an absolute value of a cathode voltage output from the bipolar high-voltage generator 100 is within a preset range.

The bipolar high-voltage generator 100 may include a first transformer T1 and a second transformer T2. The first transformer T1 is provided in an output circuit of the anode high-voltage, and the second transformer T2 is provided in an output circuit of the cathode high-voltage. In some embodiments, a working state of at least one of the first transformer T1 and the second transformer T2 is adjustable. In some embodiments, the working state may include at least one of a leakage inductance of the first transformer T1, a leakage inductance of the second transformer T2, a working frequency of the first transformer T1, or a working frequency of the second transformer T2. In some embodiments, a difference between a leakage inductance of the first transformer T1 and a leakage inductance of the second transformer T2 is a target leakage inductance difference. The target leakage inductance difference corresponds to the target voltage difference, and the target leakage inductance difference is not zero. The target voltage difference is within the preset range, and the target voltage difference is defined as a difference between an absolute value of a target anode voltage and an absolute value of a target cathode voltage output from the bipolar high-voltage generator 100. In some embodiments, the first transformer T1 or the second transformer T2 may include a primary winding and a secondary winding, at least one of a first overlap value between the primary winding and the secondary winding, a winding thickness of the primary winding, and a winding thickness of the secondary winding is adjustable. In some embodiments, the working frequency of the first transformer T1 or a working frequency of the second transformer T2 is a target working frequency, and the target working frequency corresponds to the target voltage difference.

As shown in FIG. 1, the bipolar high-voltage generator 100 may further include an inverter circuit 110, a resonant circuit 120, a first voltage multiplier rectifier circuit 130, and a second voltage multiplier rectifier circuit 140. An output of the inverter circuit 110 is connected to an input of the resonant circuit 120, and outputs of the resonant circuit 120 are connected to an input of the first transformer T1 and an input of the second transformer T2, respectively. An output of the first transformer T1 is connected to an input of the first voltage multiplier rectifier circuit 130, and an output of the first voltage multiplier rectifier circuit 130 is connected to the anode 11 of the X-ray tube 10 to output the anode high-voltage to the X-ray anode 11 of the X-ray tube 10. An output of the second transformer T2 is connected to an input of the second voltage multiplier rectifier circuit 140, and an output of the second voltage multiplier rectifier circuit 140 is connected to the cathode 12 of the X-ray tube 10 to output the cathode high-voltage to the cathode 12 of the X-ray tube 10.

The inverter circuit 110 is used to invert and convert an input direct current (DC) power source. In some embodiments, the inverter circuit 110 includes a plurality of inverter bridge arms connected in parallel. The plurality of inverter bridge arms include bridge arm switches connected in series, with a midpoint of the bridge arm switches being the output of the inverter circuit 110. As shown in FIG. 1, the inverter circuit 110 may include a first inverter bridge arm and a second inverter bridge arm connected in parallel. The first inverter bridge arm includes a first bridge arm upper switch Q1 and a first bridge arm lower switch Q3 connected in series. The second inverter bridge arm includes a second bridge arm upper switch Q2 and a second bridge arm lower switch Q4 connected in series. The inverter circuit 110 may perform inversion conversion on the input DC power source to output an alternating current (AC) power source to the resonant circuit 120.

The resonant circuit 120 is used to perform resonant conversion on the input AC power source (for example, the AC power source input from the inverter circuit 110), thereby improving power conversion efficiency. The resonant circuit 120 may include at least one of an inductor or a capacitor, such as an inductor-capacitor-capacitor (LCC) resonant circuit, or the like.

The transformers (e.g., the first transformer T1 and the second transformer T2) are used to realize a step-up of the AC power source. As shown in FIG. 1, a primary winding of the first transformer T1 and a primary winding of the second transformer T2 are connected to the outputs of the resonant circuit 120, respectively. A secondary winding of the first transformer T1 is connected to the input of the first voltage multiplier rectifier circuit 130, and a secondary winding of the second transformer T2 is connected to the input of the second voltage multiplier rectifier circuit 140.

The voltage multiplier rectifier circuits (e.g., the first voltage multiplier rectifier circuit 130 and the second voltage multiplier rectifier circuit 140) are used to realize rectification of the AC power source and amplification of the output according to a preset multiplier. In some embodiments, the voltage multiplier rectifier circuits may adopt corresponding structures such as a rectifier bridge, an amplifier circuit, or the like.

The bipolar high-voltage generator 100 may be powered by the inverter circuit 110, which is input to the first transformer T1 and the second transformer T2 via the resonant circuit 120. The AC input to the first transformer T1 outputs the anode high-voltage KV1 and an anode current mA1 to the anode 11 of the X-ray tube 10 via the first voltage multiplier rectifier circuit 130. The AC input to the second transformer T2 via the resonant circuit 120 outputs the cathode high-voltage KV2 and a cathode current mA2 to the cathode 12 of the X-ray tube 10 via the second voltage multiplier rectifier circuit 140.

In some embodiments, the first transformer T1 and the second transformer T2 may share the same magnetic core or use separate magnetic cores respectively. In some embodiments, the first transformer T1 and the second transformer T2 may share the same primary winding or include separate primary windings, respectively. That is, the cathode and the anode may be equipped with transformers that are independent of each other, or integrated transformers.

In some embodiments, the bipolar high-voltage generator includes the magnetic core, a first primary winding, a first secondary winding, and a second secondary winding. The magnetic core, the first primary winding, and the first secondary winding form the first transformer, and the magnetic core, the first primary winding, and the second secondary winding form the second transformer.

In some embodiments, the first primary winding is wound on a magnetic pillar of the magnetic core, the first secondary winding and the second secondary winding are wound side-by-side on the first primary winding, and the first secondary winding and the second secondary winding are spaced apart along an axial direction of the magnetic pillar.

In some embodiments, the first primary winding is wound on the magnetic pillar of the magnetic core, the second secondary winding is wound on the first primary winding, and the first secondary winding is wound on the second secondary winding.

In some embodiments, the first primary winding, the first secondary winding, and the second secondary winding may each include a set of windings, or may each include a plurality of sets of windings in series and parallel. In some embodiments, the magnetic core may include a U-type core, an E-type core, an RM-type core, a PQ-type core, or the like.

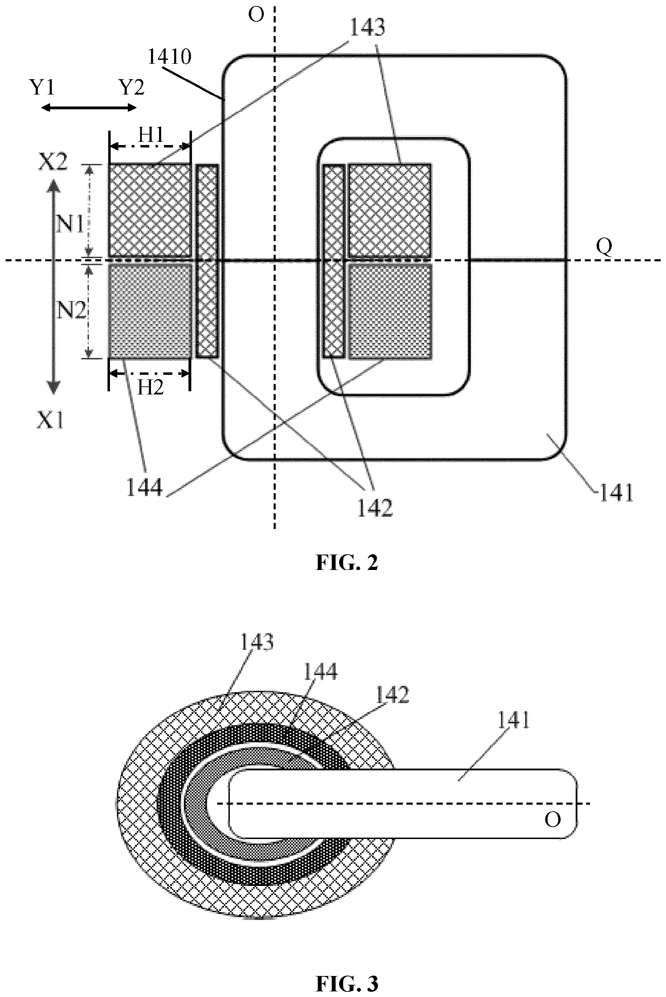

FIG. 2 is a schematic diagram illustrating a first structure of a transformer according to some embodiments of the present disclosure. As shown in FIG. 2, the bipolar high-voltage generator 100 may include a magnetic core 141, a first primary winding 142, a first secondary winding 143, and a second secondary winding 144. The first primary winding 142 is wound on a magnetic pillar 1410 of the magnetic core 141, the first secondary winding 143 and the second secondary winding 144 are wound side by side on the first primary winding 142, and the first secondary winding 143 and the second secondary winding 144 are spaced apart along the axial direction of the magnetic pillar 1411. The magnetic core 141, the first primary winding 142, and the first secondary winding 143 form the first transformer T1. The magnetic core 141, the first primary winding 142, and the second secondary winding 144 form the second transformer T2.

FIG. 3 is a schematic diagram illustrating a second structure of a transformer according to some embodiments of the present disclosure. As shown in FIG. 3, the bipolar high-voltage generator 100 may include the magnetic core 141, the first primary winding 142, the first secondary winding 143, and the second secondary winding 144. The first primary winding 142 is wound on the magnetic pillar of the magnetic core 141, the second secondary winding 144 is wound on the first primary winding 142, and the first secondary winding 143 is wound on the second secondary winding 144. The magnetic core 141, the first primary winding 142, and the first secondary winding 143 form the first transformer T1. The magnetic core 141, the first primary winding 142 and the second secondary winding 144 from the second transformer T2.

In the embodiment shown in FIGs. 2 and 3, the first transformer T1 and the second transformer T2 are integrally provided and share the same magnetic core 141 and the same primary winding (the first primary winding 142). The first primary winding 142 is connected to the output of the resonant circuit 120, the first secondary winding 143 is connected to the first voltage multiplier rectifier circuit 130, and the second secondary winding 144 is connected to the second voltage multiplier rectifier circuit 140. The first primary winding 142 and the first secondary winding 143 are coupled through the magnetic core 141 to transfer an AC power source of the output circuit of the anode high-voltage KV1, and the first primary winding 142 and the second secondary winding 144 are coupled through the magnetic core 141 to transfer an AC power source of the output circuit of the cathode high-voltage KV2. In some embodiments, the first primary winding 142, the first secondary winding 143, and the second secondary winding 144 may each include a set of windings, or may include a plurality of sets of windings in series and parallel. In some embodiments, the magnetic core 141 may be a U-type core, an E-type core, an RM-type core, a PQ-type core, or the like.

In some embodiments, the bipolar high-voltage generator includes the magnetic core, the first primary winding, a second primary winding, the first secondary winding, and the second secondary winding. The magnetic core, the first primary winding, and the first secondary winding form the first transformer, and the magnetic core, the second primary winding, and the second secondary winding form the second transformer. The first primary winding is wound on a first magnetic pillar of the magnetic core, and the first secondary winding is wound on the first primary winding. The second primary winding is wound on a second magnetic pillar of the magnetic core, and the second secondary winding is wound on the second primary winding. The first magnetic pillar is provided opposite the second magnetic pillar. In some embodiments, the first primary winding, the second primary winding, the first secondary winding, and the second secondary winding may each include a set of windings, or may each include a plurality of sets of windings in series and parallel.

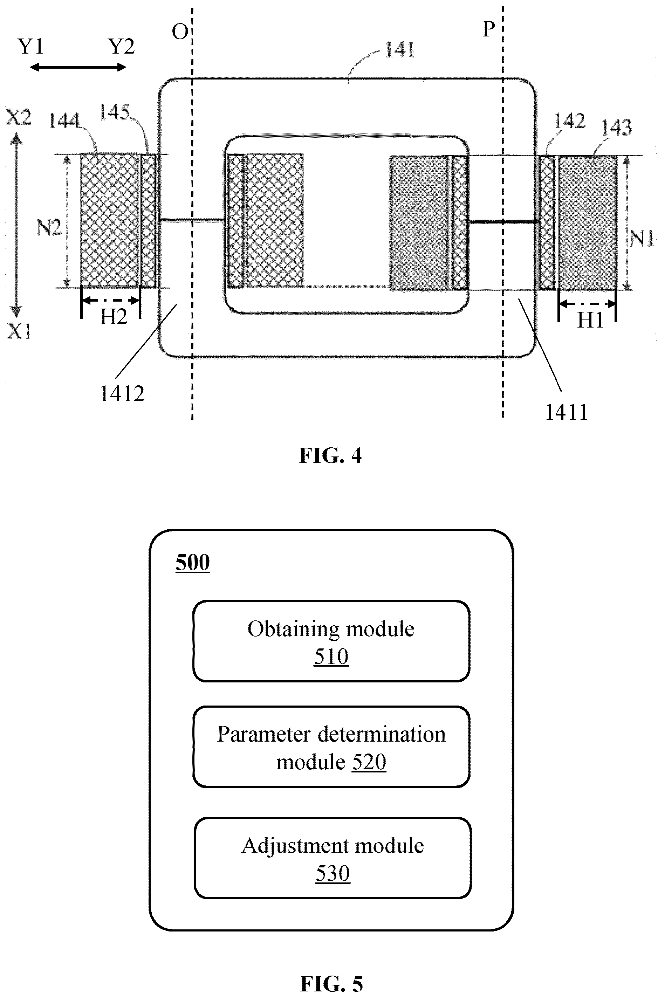

FIG. 4 is a schematic diagram illustrating a third structure of a transformer according to some embodiments of the present disclosure. As shown in FIG. 4, the bipolar high-voltage generator 100 may include the magnetic core 141, the first primary winding 142, a second primary winding 145, the first secondary winding 143, and the second secondary winding 144. The first primary winding 142 is wound on a first magnetic pillar 1411 of the magnetic core 141, the first secondary winding 143 is wound on the first primary winding 142, and the magnetic core 141, the first primary winding 142, and the first secondary winding 143 form the first transformer T1. The second primary winding 145 is wound on a second magnetic pillar 1412 of the magnetic core 141, the second secondary winding 144 is wound on the second primary winding 145, and the magnetic core 141, the second primary winding 145, and the second secondary winding 144 form the second transformer T2. The first magnetic pillar 1411 is disposed opposite the second magnetic pillar 1422. In some embodiments, the first primary winding 142, the second primary winding 145, the first secondary winding 143, and the second secondary winding 144 may each include a set of windings, respectively, or include a plurality of sets of windings connected in series and parallel. In some embodiments, the magnetic core 141 may be a U-type core, an E-type core, an RM-type core, a PQ-type core, or the like.

In the embodiment shown in FIG. 4, the first transformer T1 and the second transformer T2 are integrally set up to share the same magnetic core 141, but are wound with different primary windings (the first primary winding 142 or the second primary winding 145) at different pillar positions of the magnetic core, respectively. The first primary winding 142 and the second primary winding 145 are connected to the outputs of the resonant circuit 120, respectively, the first secondary winding 143 is connected to the first voltage multiplier rectifier circuit 130, and the second secondary winding 144 is connected to the second voltage multiplier rectifier circuit 140. The first primary winding 142 and the first secondary winding 143 are coupled through the magnetic core 141 to transfer the AC power source of the output circuit of the anode high-voltage KV1, and the second primary winding 145 and the second secondary winding 144 are coupled through the magnetic core 141 to transfer the AC power source of the output circuit of the cathode high-voltage KV2.

As shown in FIG. 1, the first transformer T1 and the first voltage multiplier rectifier circuit 130 form the output circuit of the anode high-voltage, the second transformer T2 and the second voltage multiplier rectifier circuit 140 form the output circuit of the cathode high-voltage, and the two output circuits share the inverter circuit 110 and the resonant circuit 120. According to the working principle of the LCC resonant circuit, magnitudes of the cathode high-voltage KV2 and the anode high-voltage KV1 are determined by a gain M of each of the output circuits and an inverter input voltage Vin. Assuming that the gain of the output circuit of the cathode high-voltage KV2 is M1, and that the gain of the output circuit of the anode high-voltage KV1 is M2, the anode high-voltage KV1 = M1*Vin and the cathode high-voltage KV2 = M2*Vin. Since the inverter circuit is shared, the inverter input voltages of the cathode and the anode are exactly equal, and the relative magnitudes of M1 and M2 are determined by the magnitude of the load: the lighter the load, the greater the gain M.

In conjunction with the above disclosure, the anode current of the X-ray tube is less than the cathode current due to scattered electrons, i.e., the bipolar high-voltage generator 100 operates with a lighter anode load than the cathode. If the bipolar high-voltage generator 100 only controls a total voltage to satisfy a set voltage (for example, the set voltage may be in a range of 110 kV-150 kV), the cathode high-voltage is smaller than the anode high-voltage due to the difference in load weight, which leads to an imbalance of the cathode high-voltage and the anode high-voltage. The total voltage refers to the total value of the voltages between the cathode and the anode.

The X-ray system provided in the embodiments of the present disclosure not only controls the total voltage but also ensures that the difference between the absolute value of the anode high-voltage and the absolute value of the cathode high-voltage output from the bipolar high-voltage generator 100 falls within the preset range, thereby achieving balanced control of the cathode high-voltage and the anode high-voltage

In some embodiments, the target voltage difference of the bipolar high-voltage generator 100 may be obtained to adjust a working state of at least one of the first transformer T1 and the second transformer T2 based on the target voltage difference. The target voltage difference is within the preset range. For example, the preset range is [-15 kV, +15 kV], [-12 kV, +12 kV], [-10 kV, +10 kV], [-8 kV, +8 kV], [-6 kV, + 6 kV], [-5 kV, +5kV], or the like. In some embodiments, the target voltage difference is zero. In some embodiments, the target voltage difference is greater than zero or less than zero. For example, the target voltage difference is - 10 kV, -8 kV, -5 kV, 0, 5 kV, 6 kV, or 10 kV, among other values. In some embodiments, the target voltage difference or the preset range may be determined based on at least one of a working state, a load, an insulation structure, etc., of the bipolar high-voltage generator. In some embodiments, the target voltage difference may be a preset value, or a dynamic value determined based on a real-time working state. In some embodiments, different set voltages may correspond to the same or different target voltage differences.

In some embodiments, a leakage inductance of at least one of the first transformer and the second transformer may be adjusted based on the target voltage difference to produce a target leakage inductance difference of the bipolar high-voltage generator. The target leakage inductance difference corresponds to the target voltage difference. The target leakage inductance difference is the leakage inductance difference between a target leakage inductance of the first transformer and a target leakage inductance of the second transformer. That the bipolar high-voltage generator produces a target leakage inductance difference means that the leakage inductance difference between the leakage inductance of the first transformer and the leakage inductance of the second transformer reaches the target leakage inductance difference.

In some embodiments, a user may manually adjust the leakage inductance of at least one of the first transformer and the second transformer, so that the leakage inductance difference between the leakage inductance of the first transformer and the leakage inductance of the second transformer reaches the target leakage inductance difference.

In some embodiments, the X-ray system 1000 may include an adjustment device (not shown in the figures) connected to the bipolar high-voltage generator 100. The adjustment device may be configured to obtain the target voltage difference of the bipolar high-voltage generator 100; determine an adjustment parameter based on the target voltage difference; and adjust the leakage inductance of at least one of the first transformer and the second transformer based on the adjustment parameter to make the bipolar high-voltage generator produce the target leakage inductance difference. The target leakage inductance difference corresponds to the target voltage difference. For example, the adjustment device may adjust a leakage inductance L1 of the first transformer T1, or adjust a leakage inductance L2 of the second transformer T2, or adjust both the leakage inductance L1 of the first transformer T1 and the leakage inductance L2 of the second transformer T2, to make the leakage inductance difference between the leakage inductance L1 of the first transformer T1 and the leakage inductance L2 of the second transformer T2 reach the target leakage inductance difference. In some embodiments, the adjustment device may determine a reference leakage inductance difference of the first transformer and the second transformer based on the target voltage difference; determine the adjustment parameter based on the reference leakage inductance difference; and adjust the windings of at least one of the first transformer and the second transformer based on the adjustment parameter, to make the bipolar high-voltage generator produce the target leakage inductance difference.

In some embodiments, the adjustment device may include a sampling circuit and a control circuit. The sampling circuit may be configured to sample parameters, such as the anode high-voltage, the cathode high-voltage, the input voltage of the first transformer, the input voltage of the second transformer, etc., output by the bipolar high-voltage generator. The control circuit may be configured to obtain the target voltage difference, determine the reference leakage inductance difference between the first transformer and the second transformer based on the target voltage difference, determine the adjustment parameter based on the reference leakage inductance difference, and adjust at least one of the first transformer and the second transformer based on the adjustment parameter. In some embodiments, the control circuit is configured to read a current anode high-voltage and a current cathode high-voltage output by the bipolar high-voltage generator during an adjustment process, determine whether a voltage difference between an absolute value of the current anode high-voltage and an absolute value of the current cathode high-voltage reaches the target voltage difference or falls within the preset range. In response to determining that the target voltage difference has been reached or falls within the preset range, the control circuit ends the adjustment; and in response to determining that the target voltage difference has not been reached or fall outside the preset range, the control circuit performs further fine-tuning of the leakage inductance until the voltage difference reaches the target voltage difference or falls within the preset range. For example, the control circuit may, based on the target voltage difference, further adjust at least one of the leakage inductance of the first transformer T1 or the leakage inductance of the second transformer T2, until the difference between the absolute value of the current anode high-voltage output by the bipolar high-voltage generator 100 and the absolute value of the current cathode high-voltage output by the bipolar high-voltage generator 100 reaches the target voltage difference. As another example, the control circuit may update the target voltage difference based on the preset range, the current anode high-voltage, and the current cathode high-voltage, and further adjust at least one of the leakage inductance of the first transformer T1 or the leakage inductance of the second transformer T2 based on the updated target voltage difference, to make the difference between the absolute value of the current anode high-voltage output by the bipolar high-voltage generator 100 and the absolute value of the current cathode high-voltage output by the bipolar high-voltage generator 100 fall within the preset range. In some embodiments, the adjustment device includes a mechanical mechanism. The mechanical mechanism refers to a structure for adjusting at least one of the first transformer or the second transformer. For example, the adjustment device adjusts at least one of a winding overlap value or a winding thickness of the first transformer or the second transformer through a mechanical structure such as a mechanical claw, a toggle, or the like, to realize adjustment of the leakage inductance.

In the practical application of the bipolar high-voltage generator, there is a significant leakage inductance in both the first transformer and the second transformer, and the generated leakage inductance is connected in series to the corresponding output circuits (e.g., the output circuit of the anode voltage and the output circuit of the cathode voltage) and divides the input voltage in the output circuits. An impedance value of the leakage inductance, Z=jωL, wherein ω denotes a frequency of the inverter circuit 110, j denotes an imaginary unit, and L denotes an inductance value of the leakage inductance. The larger the inductance value of the leakage inductance, the larger the impedance value of the leakage inductance, and the larger the voltage division of the divider to the input voltage. Therefore, when the input voltage and the current of the first transformer T1 and the second transformer T2 are equal, the transformer with the larger leakage inductance the first transformer T1 and the second transformer T2 has a smaller corresponding output voltage.

By adjusting the leakage inductance of at least one of the first transformer and the second transformer based on the adjustment parameter, the leakage inductance difference between the leakage inductance L1 of the first transformer T1 and the leakage inductance L2 of the second transformer T2 is made to reach the target leakage inductance difference. The leakage inductance L1 of the first transformer T1 divides the input voltage of the first transformer T1, and the leakage inductance L2 of the second transformer T2 divides the input voltage of the second transformer T2, thereby causing the output voltage of the first transformer T1 to reach a first preset voltage and the output voltage of the second transformer T2 to reach a second preset voltage. Reaching the respective preset voltages may further ensure that the anode high-voltage KV1 output to the anode 11 reaches the target anode high-voltage, the cathode high-voltage KV2 output to the cathode 12 reaches the target cathode high-voltage, and the voltage difference between the absolute value of the target anode high-voltage and the absolute value of the target cathode high-voltage reaches the target voltage difference. Reaching the target voltage difference may cause the difference between the absolute value of the anode voltage and the absolute value of the cathode voltage output by the bipolar high-voltage generator 100 to fall within the preset range.

In some embodiments, the leakage inductance of the transformer (e.g., the first transformer and the second transformer) is related to the structure and the position of the windings of the transformer. Correspondingly, the adjustment device may control the leakage inductance difference between the leakage inductance L1 of the first transformer T1 and the leakage inductance L2 of the second transformer T2 to reach the target leakage inductance difference by adjusting a parameter of the windings (e.g., the winding thickness of the windings, an overlap value between the windings, etc.) of at least one of the first transformer T1 and the second transformer T2.

In some embodiments, the adjustment parameter may include at least one of a first adjustment parameter of the first transformer and a second adjustment parameter of the second transformer. In some embodiments, the first adjustment parameter includes at least one of a first overlap value between the primary winding and the secondary winding of the first transformer, a winding thickness of the primary winding of the first transformer, and a winding thickness of the secondary winding of the first transformer. In some embodiments, the second adjustment parameter includes at least one of a second overlap value between the primary winding and the secondary winding of the second transformer, a winding thickness of the primary winding of the second transformer, and a winding thickness of the secondary winding of the second transformer.

In some embodiments, before outputting different target voltage differences, the adjustment device may determine the adjustment parameter based on the different target voltage differences through mathematical computation, simulation, or by utilizing a trained parameter determination model. For example, the adjustment device may determine at least one of a leakage inductance required to be adjusted for the first transformer or a leakage inductance required to be adjusted for the second transformer, and based on the leakage inductance, determine at least one of an overlap value or a winding thickness for a winding required to be adjusted for at least one of the first transformer or the second transformer.

In some embodiments, in the transformers shown in FIG. 2, FIG. 3, or FIG. 4, an overlap difference between a first overlap value N1 and a second overlap value N2 is a target overlap difference corresponding to the target leakage inductance difference, and/or, a thickness difference between the winding thickness of the first secondary winding 143 and the winding thickness of the second secondary winding 144 is a target thickness difference corresponding to the target leakage inductance difference, and at least one of the target overlap difference or the target thickness difference is not zero. Exemplarily, during a configuration connection, based on structures of the first transformer T1 and the second transformer T2 as shown in FIG. 4, to reach the target voltage output (e.g., the target anode high-voltage and the target cathode high-voltage) at the anode 11 and the cathode 12, thereby meeting voltage balance or different power supply requirements, at least one of the first adjustment parameter or the second adjustment parameter (e.g., the first overlap value, the second overlap value, the winding thickness of the first transformer, and the winding thickness of the second transformer) may be determined through mathematical computation, simulation, or the trained machine learning model. When adjusting the leakage inductance of the first transformer T1, the first overlap value N1 or the winding thickness of the first secondary winding 143 of the first transformer T1 is adjusted based on the first adjustment parameter. Correspondingly, when adjusting the leakage inductance of the second transformer T2, the second overlap value N2 of the second transformer T2 or the winding thickness of the second secondary winding 144 is adjusted based on the second adjustment parameter. Thus, through the aforementioned adjustments, the corresponding target leakage inductance difference may be adjusted, ensuring that the overlap difference between the first transformer T1 and the second transformer T2 at the completion of the configuration corresponds to the target overlap difference for the target leakage inductance difference, and/or the thickness difference between the winding thickness of the first secondary winding 143 and the winding thickness of the second secondary winding 144 corresponds to the target thickness difference for the target leakage inductance difference. When configuring the connection, the overlap value and the winding thickness may be adjusted at the same time, or one of the parameters may be fixed and the other may be adjusted in any specific manner.

In some embodiments, the adjustment device may be a structure that is independent of the X-ray system. In such cases, the adjustment device may be used to adjust the leakage inductance of at least one of the first transformer and the second transformer when leakage inductance adjustment is required.

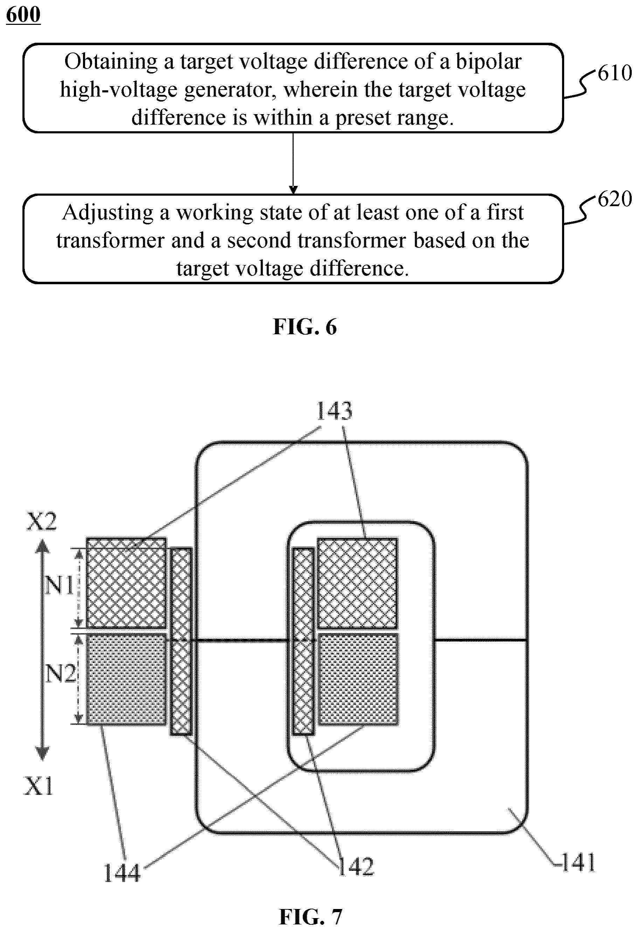

More details regarding the leakage inductance adjustment of the transformer may be found in FIG. 6 and its related description.

In some embodiments, the working frequency of at least one of the first transformer T1 or the second transformer T2 may be adjusted, based on the target voltage difference, by the inverter circuit 110, ensuring that the difference between the absolute value of the anode voltage output by the bipolar high-voltage generator 100 and the absolute value of the cathode voltage output by the bipolar high-voltage generator 100 falls within the preset range.

In some embodiments, the X-ray system 1000 includes a control device (not shown in the figures) connected to the bipolar high-voltage generator. The control device may be a central processor, a control circuit, a host computer (e.g., a host computer connected to the X-ray system), etc., which is not limited in the present disclosure. The control device may be configured to obtain the target voltage difference of the bipolar high-voltage generator 100, adjust the working frequency of at least one of the first transformer and the second transformer based on the target voltage difference through the inverter circuit 110, to make the difference between the absolute value of the anode voltage output by the bipolar high-voltage generator 100 and the absolute value of the cathode voltage output by the bipolar high-voltage generator 100 fall within the preset range. In some embodiments, the control device may adjust the working frequency of at least one of the first transformer or the second transformer by the inverter circuit 110, to increase the gain of the cathode voltage output by the bipolar high-voltage generator, and decrease the gain of the anode voltage.

In some embodiments, the adjustment device and the control device may be mutually independent modules in the bipolar high-voltage generator, or integrated together. For example, the control device and the adjustment device may be a single module including the sampling circuit and the control circuit. The sampling circuit is used to sample the parameters such as the anode high-voltage, the cathode high-voltage, the input voltage of the first transformer T1, the input voltage of the second transformer T2, or the like, output by the bipolar high-voltage generator 100. The control circuit may be used to obtain the target voltage difference, adjust the working state (e.g., the leakage inductance or the working frequency) of at least one of the first transformer T1 and the second transformer T2 based on the target voltage difference, to make that the difference between the absolute value of the anode voltage and the absolute value of the cathode voltage output from the bipolar high-voltage generator 100 falls within the preset range.

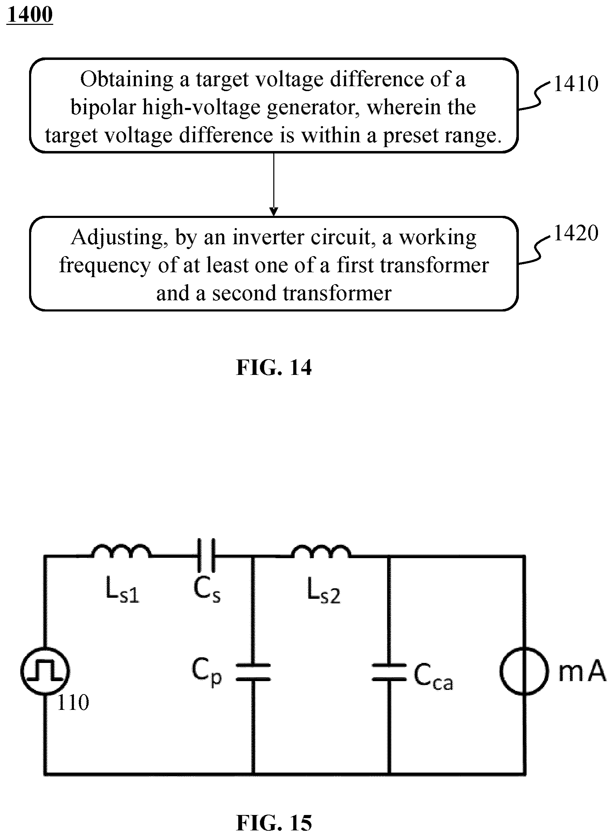

More details regarding the adjustment of the working frequency of the transformer may be found elsewhere in the present disclosure, such as FIG. 14 and its related descriptions.

The X-ray system 1000 provided in the embodiments of the present disclosure, achieves the goal of balancing the output voltage by ensuring that the difference between the absolute value of the anode high-voltage and the absolute value of the cathode high-voltage output by the bipolar high-voltage generator falls within the preset range ( the preset range including zero), can set the target voltage difference to zero or another value close to zero within the preset range. The X-ray system 1000 can make the voltage difference of the output of the bipolar high-voltage generator reach the target voltage difference by performing the adjustment of the working state of at least one of the first transformer T1 or the second transformer T2, thereby achieving the purpose of balancing the output voltage. The method eliminates the need for additional inverter circuit structures, simplifies the structures of the bipolar high-voltage generator 100 and the X-ray system 1000, reduces the design cost, and increases the system reliability.

It should be noted that the above description of the X-ray system 100 and its components (e.g., the bipolar high-voltage generator 100, the first transformer T1, the second transformer T2, etc.) is provided for descriptive convenience only, and does not limit the present disclosure to the scope of the cited embodiments. It should be appreciated that for a person skilled in the art, after comprehending the principles of the system, it is possible to combine various modules in any manner or form subsystems connected with other modules without departing from the principles. Deformations such as these are within the scope of protection of the present disclosure.

FIG. 5 is a diagram illustrating an exemplary module of a control system of a bipolar high-voltage generator according to some embodiments of the present disclosure. As shown in FIG. 5, in some embodiments, a control system 500 may include an obtaining module 510, a parameter determination module 520, and an adjustment module 530. In some embodiments, the control system 500 may be an adjustment device or a control device, or a collection of the adjustment device and the control device, as described above for the bipolar high-voltage generator 100. For example, the adjustment device and the control device may be of a same structure, both including the obtaining module 510, the parameter determination module 520, and the adjustment module 530. As another example, the adjustment device includes the obtaining module 510, the parameter determination module 520, and the adjustment module 530, and the control device includes the obtaining module 510 and the adjustment module 530.

The obtaining module 510 may obtain a target voltage difference of the bipolar high-voltage generator, wherein the target voltage difference is within a preset range.

The parameter determination module 520 may determine an adjustment parameter based on the target voltage difference. The adjustment parameter may include at least one of a first adjustment parameter of a first transformer and a second adjustment parameter of a second transformer. In some embodiments, the first adjustment parameter includes at least one of a first overlap value between a primary winding and a secondary winding of the first transformer, a winding thickness of the primary winding of the first transformer, and a winding thickness of the secondary winding of the first transformer. The second adjustment parameter includes at least one of a second overlap value between the primary winding and the secondary winding of the second transformer, a winding thickness of the primary winding of the second transformer, and a winding thickness of the secondary winding of the second transformer. The first overlap value is a dimensional overlap value between the primary winding and the secondary winding of the first transformer along an axial direction of a first magnetic pillar, and the second overlap value is a dimensional overlap value between the primary winding and the secondary winding of the second transformer along an axial direction of a second magnetic pillar. The winding thickness is a dimension of the overall winding along a radial direction of a magnetic pillar after winding.

In some embodiments, the parameter determination module 520 determines at least one of the first adjustment parameter or the second adjustment parameter based on the target voltage difference by mathematical computation, simulation, or a trained parameter determination model.

In some embodiments, the parameter determination module 520 determines a reference leakage inductance difference of the first transformer and the second transformer based on the target voltage difference, and determines at least one of the first adjustment parameter or the second adjustment parameter based on the reference leakage inductance difference.

The adjustment module 530 may adjust a working state of at least one of the first transformer and the second transformer based on the target voltage difference or the adjustment parameter, so that a difference between an absolute value of an anode voltage and an absolute value of a cathode voltage output from the bipolar high-voltage generator falls within the preset range.

In some embodiments, the adjustment module 530 performs at least one of the following adjustment operations to make the bipolar high-voltage generator produce a target leakage inductance difference, the target leakage inductance difference corresponding to the target voltage difference: adjusting the first overlap value based on the first adjustment parameter, adjusting the second overlap value based on the second adjustment parameter, adjusting the winding thickness of the first secondary winding based on the first adjustment parameter, and adjusting the winding thickness of the second secondary winding based on the second adjustment parameter.

In some embodiments, the adjustment module 530 performs at least one of the following operations to make an overlap difference between the first overlap value and the second overlap value correspond to the target leakage inductance difference, and the overlap difference is not zero: adjusting the first overlap value based on the first adjustment parameter, and adjusting the second overlap value based on the second adjustment parameter.

In some embodiments, the adjustment module 530 performs at least one of the following operations to make a thickness difference between the winding thickness of the first secondary winding and the winding thickness of the second secondary winding correspond to the target leakage inductance difference, and the thickness difference is not zero: adjusting the winding thickness of the first secondary winding based on the first adjustment parameter, and adjusting the winding thickness of the second secondary winding based on the second adjustment parameter.

In some embodiments, the adjustment module 530 adjusts, by an inverter circuit, a working frequency of at least one of the first transformer and the second transformer based on the target voltage difference, to make the difference between the absolute value of the anode voltage and the absolute value of the cathode voltage output from the bipolar high-voltage generator fall within the preset range. In some embodiments, the adjustment module 530 adjusts, by the inverter circuit, the working frequency of at least one of the first transformer and the second transformer via the inverter circuit, to increase the gain of the cathode voltage output by the bipolar high-voltage generator, and decrease the gain of the anode voltage output by the bipolar high-voltage generator.

More details regarding the obtaining module 510, the parameter determination module 520, and the adjustment module 530 may be found in FIG. 6 and its related description.

It should be noted that the above description of the control system 500 and its modules is provided only for descriptive convenience, and does not limit the present disclosure to the scope of the cited embodiments. It should be appreciated that for a person skilled in the art, after comprehending the principles of the system, it is possible to combine various modules in any manner, or form subsystems connected with other modules without departing from the principles. In some embodiments, the obtaining module 510, the parameter determination module 520, and the adjustment module 530 disclosed in FIG. 5 may be different modules in a single system, or a single module realizing the aforementioned functions of two or more modules. For example, the individual modules may share a common storage module, and the individual modules may each have a respective storage module. Morphisms such as these are within the scope of protection of the present disclosure.

FIG. 6 is an exemplary flowchart illustrating a method of controlling a bipolar high-voltage generator according to some embodiments of the present disclosure. As shown in FIG. 6, process 600 includes the following operations. In some embodiments, the process 600 may be performed by the control system 500.

In 610, a target voltage difference of a bipolar high-voltage generator may be obtained. In some embodiments, operation 610 is performed by the obtaining module 510.

The target voltage difference refers to a voltage difference between an absolute value of a target anode high-voltage output by the bipolar high-voltage generator and an absolute value of a target cathode high-voltage output by the bipolar high-voltage generator.

The target anode high-voltage refers to a voltage value expected to be output at an anode. The target cathode high-voltage refers to a voltage value expected to be output at a cathode. In some embodiments, a total voltage value of the target anode high-voltage and the target cathode high-voltage satisfies a set voltage (e.g., the set voltage may be in a range of 110 kV to 150 kV), i.e., a total voltage between the anode and the cathode satisfies the set voltage. For example, the set voltage is 110 kV, the anode high-voltage is 60 kV, and the cathode high-voltage is -50 kV; or the anode high-voltage is 110 kV, and the cathode high-voltage is 0 kV; or the anode high-voltage is 0kV, and the cathode high-voltage is -110 kV; or the anode high-voltage is 55 kV, and the cathode high-voltage is -55 kV.

The target voltage difference is within a preset range, e.g., at [-15 kV, +15 kV], [-12 kV, +12 kV], [-10 kV, +10 kV], [-8 kV, +8 kV], [-6 kV, +6 kV], [-5 kV, +5 kV], and other ranges. In some embodiments, the preset range may be determined based on at least one of working parameters (e.g., the set voltage, real-time output of the anode high-voltage and the cathode high-voltage, etc.), a load (e.g., an X-ray tube), an insulation structure (e.g., insulation requirements, insulation involved), etc., of the bipolar high-voltage generator. In some embodiments, the preset range may be determined based on historical data or historical experience. For example, a staff member may manually set the preset range based on experience. As another example, the control system 500 may automatically determine the preset range based on historical experimental data or historical usage data of the bipolar high-voltage generator.

In some embodiments, the preset range may be a pre-set range or a dynamic range determined based on real-time working states. In some embodiments, different set voltages may correspond to the same or different preset ranges.

In some embodiments, the target voltage difference may be any value or range within the preset range. For example, the target voltage difference is [-10 kV, + 10kV], [-8 kV, +8 kV], [-6 kV, +6 kV], or [-5 kV, +5 kV]. As another example, the target voltage difference is a value of -5 kV, 0, 5 kV, 10 kV, or the like. In some embodiments, different set voltages may correspond to the same or different target voltage differences. In some embodiments, the target voltage difference may be determined in real time based on at least one of the working parameters, the load (e.g., the X-ray tube 10), the insulation structure, etc., of the bipolar high-voltage generator (e.g., the bipolar high-voltage generator 100).

Exemplarily, when it is necessary to achieve the purpose of balancing the anode high-voltage and the cathode high-voltage output from the bipolar high-voltage generator, the control system 500 may set the target voltage difference to zero or to a value close to zero within the preset range, and, by adjusting the working state (e.g., at least one of the leakage inductance or the working frequency of the transformer) of at least one of the first transformer T1 or the second transformer T2, to make the difference between the absolute value of the anode voltage output by the bipolar high-voltage generator and the absolute value of the cathode voltage output by the bipolar high-voltage generator be the target voltage difference.

As another example, when it is desired that the difference between the absolute value of the anode voltage output by the bipolar high-voltage generator and the absolute value of the cathode voltage output by the bipolar high-voltage generator fall within the preset range [4 kV, 6 kV], the control system 500 may set the target voltage difference to 4 kV, 5 kV or 6 kV, and by adjusting the working state of at least one of the first transformer T1 or the second transformer T2, to make the difference between the absolute value of the anode voltage output by the bipolar high-voltage generator and the absolute value of the cathode voltage output by the bipolar high-voltage generator reach the target voltage difference.

In some embodiments, the control system 500 may obtain the target voltage difference or the preset range prior to configuring the connection to the bipolar high-voltage generator, during a configuration process, or afterwards. For example, the bipolar high-voltage generator 100 is configured to connect the cathode 12 and the anode 11 of the X-ray tube 10, to supply the cathode high-voltage KV2 to the cathode 12 of the X-ray tube 10, and to supply the anode high-voltage KV1 to the anode 11 of the X-ray tube 10, the first transformer T1 is provided in an output circuit of the anode high-voltage KV1, the second transformer T2 is provided in an output circuit of the cathode high-voltage KV2, and the control system 500 obtains the target voltage difference after the configuration is completed.

In some embodiments, the obtaining module 510 may first obtain the preset range, and determine the target voltage difference based on the preset range. In some embodiments, the obtaining module 510 may directly obtain the target voltage difference within the preset range.

In 620, a working state of at least one of a first transformer and a second transformer may be adjusted based on the target voltage difference. In some embodiments, operation 620 is performed by the adjustment module 530.

In some embodiments, the working state of the transformer includes at least one of the leakage inductance or the working frequency of the transformer.

In some embodiments, the adjustment module 530 may adjust the leakage inductance of at least one of the first transformer and the second transformer based on the target voltage difference. In some embodiments, the adjustment module 530 may adjust the working frequency of at least one of the first transformer and the second transformer based on the target voltage difference. In some embodiments, the adjustment module 530 may adjust the leakage inductance of at least one of the first transformer and the second transformer, and adjust the working frequency of at least one of the first transformer and the second transformer, based on the target voltage difference.

In some embodiments, the control system 500 may determine the adjustment parameter based on the target voltage difference, and adjust the working state of at least one of the first transformer and the second transformer based on the adjustment parameter.

The adjustment parameter refers to a parameter used to adjust at least one of a structure or the working state of the bipolar high-voltage generator.

In conjunction with the foregoing, the bipolar high-voltage generator includes the first transformer and the second transformer. Correspondingly, the adjustment parameter may include at least one of a first adjustment parameter of the first transformer and a second adjustment parameter of the second transformer.

In some embodiments, the first adjustment parameter includes at least one of a winding parameter or the working frequency of the first transformer, and the second adjustment parameter includes at least one of a winding parameter or the working frequency of the second transformer.

The winding parameters of the transformer may include a winding thickness of windings, an overlap value between the windings, or the like. The winding parameters affect the leakage inductance of the transformer.

In some embodiments, the first adjustment parameter includes at least one of a first overlap value between a primary winding and a secondary winding of the first transformer, a winding thickness of the primary winding of the first transformer, and a winding thickness of the secondary winding of the first transformer. For example, the first adjustment parameter is the first overlap value between the primary winding and the secondary winding of the first transformer, or the winding thickness of the primary winding of the first transformer, or the winding thickness of the secondary winding of the first transformer. As another example, the first adjustment parameter is the first overlap value between the primary winding and the secondary winding of the first transformer, and the winding thickness of the secondary winding of the first transformer.

In some embodiments, the second adjustment parameter includes at least one of a second overlap value between a primary winding and a secondary winding of the second transformer, a winding thickness of the primary winding of the second transformer, and a winding thickness of the secondary winding of the second transformer. For example, the second adjustment parameter is the second overlap value between the primary winding and the secondary winding of the second transformer, or the winding thickness of the primary winding of the second transformer, or the winding thickness of the secondary winding of the second transformer. As another example, the second adjustment parameter is the second overlap value between the primary winding and the secondary winding of the second transformer, and the winding thickness of the secondary winding of the second transformer.

The overlap value refers to a dimensional overlap value of the primary winding and the secondary winding of the transformer along the axis direction of a magnetic pillar wound thereon. Correspondingly, the first overlap value is a dimensional overlap value of the primary winding and the secondary winding of the first transformer along an axial direction of a first magnetic pillar wound therein, and the second overlap value is a dimensional overlap value of the primary winding and the secondary winding of the second transformer along an axial direction of a second magnetic pillar wound therein.

The axial direction refers to a direction of a center axis of the magnetic pillar, or a direction parallel to the center axis. For example, in conjunction with FIG. 2, the axial direction of the magnetic pillar 1410 is a direction parallel to a center axis O, such as an X1 or X2 direction in the figure.

In some embodiments, the first magnetic pillar and the second magnetic pillar may be a same magnetic pillar (e.g., the magnetic pillar 1410 shown in FIG. 2), or different magnetic pillars of a same magnetic core (e.g., the first magnetic pillar 1411 and the second magnetic pillar 1412 in FIG. 4), or different magnetic pillars of different magnetic cores (e.g., the first transformer and the second transformer are set independently of each other).

For example, as shown in FIG. 2, the first overlap value may be the dimensional overlap value N1 of the first primary winding 142 and the first secondary winding 143 of the first transformer T1 along the axial direction (e.g., in the X2 direction in the figure) of the magnetic pillar 1410, and the second overlap value is the dimensional overlap value N2 of the first primary winding 142 and the second secondary winding 144 of the second transformer T2 along the axial direction (e.g., in the X1 direction in the figure) of the magnetic pillar 1410. As another example, as shown in FIG. 4, the first overlap value may be the dimensional overlap value N1 of the first primary winding 142 and the first secondary winding 143 of the first transformer T1 along the axial direction (e.g., in a direction in which a dashed line P is located) of the first magnetic pillar 1411, and the second overlap value is the dimensional overlap value N2 of the first primary winding 145 and the second secondary winding 144 of the second transformer T2 along the axial direction (in a direction in which the dashed line P is located) of the second magnetic pillar 1412.

The winding thickness refers to, after winding, a dimension of the overall winding in a radial direction along the magnetic pillar wound. The radial direction of the magnetic pillar refers to a direction perpendicular to the axial direction. For example, in conjunction with FIG. 2, the radial direction of the magnetic pillar 1410 is a direction perpendicular to a center axis O, such as a Y1 or Y2 direction in the figure.

For example, as shown in FIG. 2, the winding thickness of the first secondary winding 143 is, a dimension H1 of the second secondary winding along the radial direction (e.g., the Y2 direction in the figure) of the magnetic pillar 1410; the winding thickness of the second secondary winding 144 is, a dimension H2 of the second secondary winding along the radial direction (e.g., the Y2 direction in the figure) of the magnetic pillar 1410.

As another example, as shown in FIG. 4, the winding thickness of the first secondary winding 143 is, the dimension H1 of the first secondary winding 143 along a radial direction (e.g., in the Y1 direction in the figure) of the first magnetic pillar 1411, and the winding thickness of the second secondary winding 144 is, the dimension H2 of the second secondary winding 144 along the radial direction (e.g., in the Y2 direction in the figure) of the second magnetic pillar 1412.

When the bipolar high-voltage generator includes the magnetic core, the first primary winding, the first secondary winding, and the second secondary winding, the magnetic core, the first primary winding, and the first secondary winding form the first transformer, and the magnetic core, the first primary winding, and the second secondary winding form the second transformer. That is, when the first transformer and the second transformer share the same magnetic core and the same primary winding (e.g., a structure of the transformer shown in FIG. 2 or FIG. 3), the first adjustment parameter may include at least one of the first overlap value between the first secondary winding and the first primary winding, and the winding thickness of the first secondary winding. Correspondingly, the second adjustment parameter may include at least one of the second overlap value between the second secondary winding and the first primary winding, and the winding thickness of the second secondary winding.

For example, in conjunction with what is shown in FIG. 2, the first adjustment parameter includes at least one of the first overlap value N1 between the first secondary winding 143 and the first primary winding 142, or the winding thickness H1 of the first secondary winding 143; the second adjustment parameter includes at least one of the second overlap value N2 between the second secondary winding 144 and the first primary winding 142, or the winding thickness H2 of the second secondary winding 144.

When the bipolar high-voltage generator includes the magnetic core, the first primary winding, a second primary winding, the first secondary winding, and the second secondary winding, i.e., when the first transformer and the second transformer share the same magnetic core but use different primary windings, the first adjustment parameter may include at least one of the first overlap value between the first secondary winding and the first primary winding along the axial direction of the first magnetic pillar, and the winding thickness of the first secondary winding. Correspondingly, the second adjustment parameter may include at least one of the second overlap value between the second secondary winding and the second primary winding along the axial direction of the second magnetic pillar, and the winding thickness of the second secondary winding.

For example, in conjunction with what is shown in FIG. 4, the first adjustment parameter includes at least one of the first overlap value N1 between the first secondary winding 143 and the first primary winding 142, or the winding thickness H1 of the first secondary winding 143; and the second adjustment parameter includes at least one of the second overlap value N2 between the second secondary winding 144 and the second primary winding 145, or the winding thickness H2 of the second secondary winding 144.

When the winding thickness of the primary winding and the secondary winding of the transformer is constant, the greater the dimensional overlap value between the primary winding and the secondary winding along the axial direction of the magnetic pillar wound, the better the coupling effect between the primary winding and the secondary winding of the transformer is, and the smaller the leakage inductance of the transformer; the smaller the dimensional overlap value between the primary winding and the secondary winding along the axial direction of the magnetic pillar wound, the worse the coupling effect between the primary winding and the secondary winding of the transformer is, and the larger the leakage inductance of the transformer.

When the overlap value between the primary winding and the secondary winding of the transformer is constant, the greater the winding thickness of at least one of the primary winding or the secondary winding, the farther the distance between the primary winding and the secondary winding, the worse the coupling effect, and the greater the leakage inductance; and the smaller the winding thickness of at least one of the primary winding or the secondary winding, the closer the distance between the primary winding and the secondary winding, the better the coupling effect, and the smaller the leakage inductance. The distance between the primary winding and the secondary winding refers to a distance between a side of the primary winding that is away from the magnetic pillar on which it is wound, and a side of the secondary winding that is away from the magnetic pillar on which it is wound, both being on a same side of the magnetic pillar. For example, as shown in conjunction with FIG. 2, the distance between the first primary winding 142 and the first secondary winding 143 is a distance between the side of the first primary winding 142 away from the magnetic pillar 1410, and the side of the first secondary winding 143 away from the magnetic pillar 1410, on a left (or right) side of the magnetic pillar 1410.

In a fixed-frequency-controlled bipolar high-voltage generator, a gain of the output voltage exhibits a second-order fluctuation characteristic with respect to the working frequency for a fixed load. Based on the second-order fluctuation characteristic, by adjusting the working frequency of the transformer, it is possible to reduce the gain of the anode voltage and increase the gain of the cathode voltage, thereby balancing the cathode voltage and the anode voltage or ensuring that the difference between the absolute value of the anode voltage and the absolute value of the cathode voltage remains within the preset range. More details regarding the working frequency may be found in FIG. 14 and its related description.

In some embodiments, the parameter determination module 520 may determine at least one of the first adjustment parameter or the second adjustment parameter based on the target voltage difference. For example, the parameter determination module 520 may determine the first adjustment parameter and the second adjustment parameter, or determine the first adjustment parameter, or determine the second adjustment parameter, based on the target voltage difference.

In some embodiments, the parameter determination module 520 may determine an adjustment mode and the corresponding adjustment parameter based on the target voltage difference. The adjustment mode refers to adjusting the first transformer or the second transformer. For example, the adjustment mode may include adjusting the first transformer, or adjusting the second transformer, or adjusting both the first transformer and the second transformer. Correspondingly, the adjustment parameter refers to an adjustment parameter corresponding to the adjustment mode. For example, if the adjustment mode is to adjust the first transformer, the corresponding adjustment parameter is the first adjustment parameter; if the adjustment mode is to adjust the second transformer, the corresponding adjustment parameter is the second adjustment parameter; if the adjustment mode is to adjust both the first transformer and the second transformer, the corresponding adjustment parameters are the first adjustment parameter and the second adjustment parameter.

In some embodiments, the determined adjustment parameter may include a specific amount of adjustment (an attenuation amount or an incremental amount) or a value to be achieved (a target value to be adjusted to). For example, it is determined that the adjustment mode is to adjust the first transformer, the corresponding first adjustment parameter includes: decreasing the first overlap value by ΔN, while increasing the winding thickness of the first secondary winding 143 by ΔH; or decreasing the first overlap value by ΔN only; or increasing the winding thickness of the first secondary winding 143 by ΔH. As another example, it is determined that the adjustment mode is to adjust the second transformer, the corresponding second adjustment parameter includes: a numerical value of the second overlap value, and a numerical value of the winding thickness of the second secondary winding 144. As another example, it is determined that the adjustment mode is to adjust the second transformer, the corresponding second adjustment parameter includes: a target working frequency of the second transformer.

In some embodiments, the parameter determination module 520 may determine at least one of the first adjustment parameter or the second adjustment parameter based on the target voltage difference by simulation, mathematical computation, or by utilizing a trained parameter determination model.

In some embodiments, an input of the parameter determination model is the target voltage difference. In some embodiments, the input of the parameter determination model includes the target voltage difference and a circuit model of the bipolar high-voltage generator (which may contain a circuit structure, a circuit parameter, and a transformer structure).

In some embodiments, an output of the parameter determination model includes at least one of the first adjustment parameter or the second adjustment parameter. In some embodiments, the output of the parameter determination model includes at least one of a parameter type or an adjustment amount corresponding to at least one of the first adjustment parameter or the second adjustment parameter. In some embodiments, the output of the parameter determination model may include the adjustment mode and the corresponding adjustment parameter.

In some embodiments, the parameter determination model includes a deep learning model such as convolutional neural networks (CNN), recurrent neural networks (RNN), generative adversarial networks (GAN), etc.

In some embodiments, the parameter determination model may be obtained by training an initial deep learning model. Exemplarily, the control system 500 may obtain historical adjustment data (including at least one of adjustment data or experimental data during actual use) of the bipolar high-voltage generator, using the historical adjustment data as a training sample (also referred to as a first training sample). The historical adjustment data includes adjustment modes, parameter types, and adjustment amounts corresponding to at least one of the different sample target voltage differences or different sample circuit models (the circuit models for different bipolar high-voltage generators), at least one of each of the sample target voltage differences or the sample circuit models including a plurality of sets of different adjustment data. For example, the different adjustment data includes different adjustment modes, different parameter types, and different adjustment amounts. At least one of an optimal adjustment mode, an optimal parameter type, or an optimal adjustment amount corresponding to at least one of each of the sample target voltage differences or the sample circuit models may be labeled manually or automatically by the system (e.g., by statistical analysis, analog simulation, etc.). The control system 500 may iteratively train the initial deep learning model using the training sample as a training input and the markers as a training output, thereby obtaining a trained parameter determination model.

In some embodiments, the control system 500 may update the parameter determination model periodically or irregularly based on actual adjustment data of the bipolar high-voltage generator. For example, the control system 500 updates the parameter determination model utilizing historical adjustment data within a preset time period from a current time to improve the accuracy of the parameter determination model.

In conjunction with the above, when the winding thickness of the transformer is fixed, the overlap value is negatively related to the leakage inductance; when the overlap value is fixed, the winding thickness is positively related to the leakage inductance. Based on the foregoing relationships, in some embodiments, when different target voltage differences need to be output, the parameter determination module 520 may determine a target leakage inductance of the first transformer and a target leakage inductance of the second transformer, by performing a circuit simulation (e.g., by a mutlsim simulation software, an Ansys simulation software, etc.) or mathematical computation of the bipolar high-voltage generator, based on the target voltage difference and relevant information of the bipolar high-voltage generator (e.g., the circuit parameter and the circuit structure). The parameter determination module 520 may determine a numerical value of at least one of the first overlap value or the winding thickness of the first transformer based on the target leakage inductance of the first transformer and the target leakage inductance of the second transformer, and/or determine a numerical value of at least one of the second overlap value or the winding thickness of the second transformer.

In some embodiments, the parameter determination module 520 may determine a reference leakage inductance difference of the first transformer and the second transformer based on the target voltage difference, and determine at least one of the first adjustment parameter and the second adjustment parameter based on the reference leakage inductance difference. In some embodiments, the parameter determination module 520 may determine, based on the reference leakage inductance difference, the adjustment mode and the corresponding adjustment parameter.

Exemplarily, after obtaining the target voltage difference and an initial anode high-voltage KV1 and an initial cathode high-voltage KV2 of the bipolar high-voltage generator 100, the parameter determination module 520 may combine the target voltage difference and the circuit parameter of the bipolar high-voltage generator 100 to build a circuit module of the bipolar high-voltage generator 100 corresponding to the circuit parameter. The parameter determination module 520 may also determine the reference leakage inductance L1 of the first transformer T1, the reference leakage inductance L2 of the second transformer T2, and the reference leakage inductance difference between the first transformer and the second transformer based on the built circuit module, by combining a structure model of the first transformer and the second transformer, determine a magnitude required to be adjusted for the leakage inductance of the first transformer and the second transformer, thereby determining at least one of the first adjustment parameter or the second adjustment parameter.

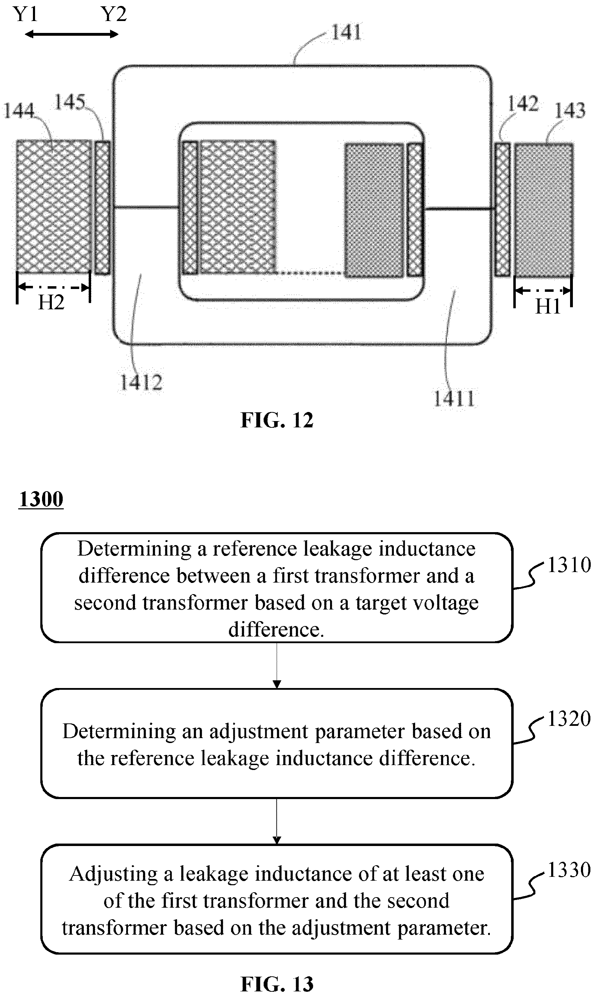

More details regarding determining the adjustment parameter based on the reference leakage inductance difference may be found in the description in FIG. 13.

In some embodiments, the adjustment module 530 may adjust the leakage inductance of at least one of the first transformer and the second transformer based on the adjustment parameter.

In some embodiments, adjusting the leakage inductance of at least one of the first transformer and the second transformer based on the adjustment parameter includes: adjusting the winding of at least one of the first transformer and the second transformer based on the adjustment parameter to make the bipolar high-voltage generator produce the target leakage inductance difference. In some embodiments, the target leakage inductance difference is not zero.

In conjunction with the above, the target leakage inductance difference refers to a difference between the target leakage inductance of the first transformer and the target leakage inductance of the second transformer. The target leakage inductance of the first transformer and the target leakage inductance of the second transformer are capable of making the output voltage of the first transformer to reach a first preset voltage and the output voltage of the second transformer to reach a second preset voltage, thereby making that the anode high-voltage output to the anode to reach the target anode high-voltage, and the cathode high-voltage of the cathode to reach the target cathode high-voltage. And the difference between the absolute value of the target anode high-voltage and the absolute value of the target cathode high-voltage reaches the target voltage difference, ensuring that the difference between the absolute value of the anode voltage output from the bipolar high-voltage generator and the absolute value of the cathode voltage output from the bipolar high-voltage generator falls within the preset range.

Exemplarily, after determining the first adjustment parameter of the first transformer T1 and the second adjustment parameter of the second transformer T2, in an actual adjustment process, a circuit connection of the first transformer T1 and the second transformer T2 may be disconnected first, and actual structural parameters of the first transformer T1 and the second transformer T2 may be adjusted by manual adjustment or automatic adjustment by the adjustment module 530, to the determined first adjustment parameter and the second adjustment parameter, respectively, and the circuit connection of the first transformer T1 and the second transformer T2 may be re-built after the adjustment is completed. In some embodiments, after completing the circuit connection, the adjustment module 530 may read (e.g., via a sampling circuit) a current anode high-voltage KV1 and a current cathode high-voltage KV2, determine the absolute value of the anode high-voltage KV1 and the absolute value of the cathode high-voltage KV2, and determine whether the voltage difference between the absolute value of the anode high-voltage KV1 and the absolute value of the cathode high-voltage KV2 reaches the target voltage difference. In response to determining that the target voltage difference is reached, the adjustment module 530 completes commissioning of the transformer; and in response to determining that the target voltage difference is not reached, the adjustment module 530 performs further fine-tuning until the voltage difference reaches the target voltage difference. Through the foregoing operations, it is possible to make the target anode high-voltage and the target cathode high-voltage of a final output stabilize in a range of the target voltage difference, and to achieve the purpose of balancing the output voltage and outputting the desired target voltage difference.

In some embodiments, the target leakage inductance of the first transformer T1 is greater than the target leakage inductance of the second transformer T2, or the target leakage inductance of the first transformer T1 is less than the target leakage inductance of the second transformer T2. Correspondingly, the target leakage inductance difference may be greater than zero or less than zero. An exact magnitude of the target leakage inductance and the target leakage inductance difference may be determined based on the target voltage difference to be output.

In some embodiments, structures of the first transformer T1 and the second transformer T2 inside the bipolar high-voltage generator 100 in an initial configuration may be the same or different. That is, the leakage inductance L1 of the first transformer T1 and the leakage inductance L2 of the second transformer T2 under the initial configuration may be equal or unequal, and the difference between the leakage inductance L1 and the leakage inductance L2 in an initial state may or may not be zero.

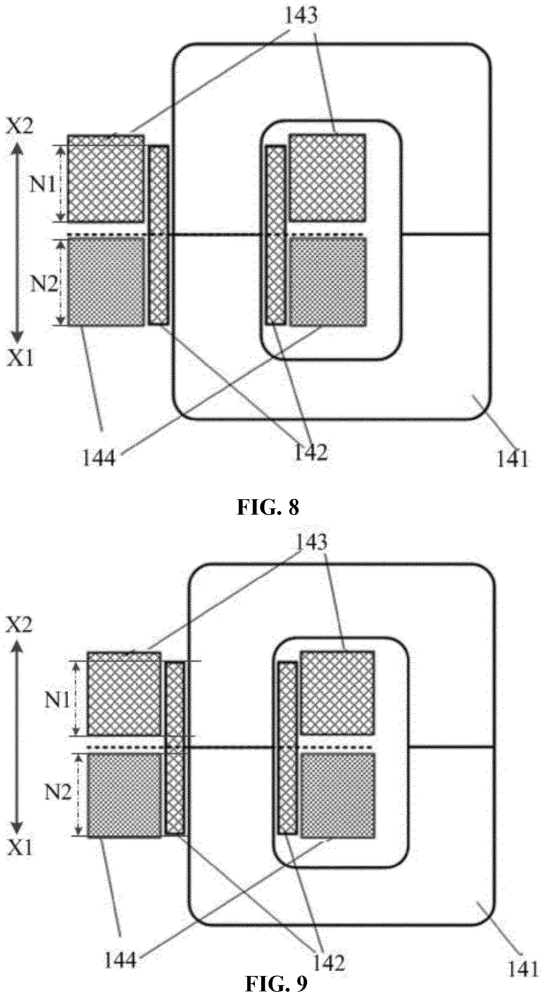

For example, in conjunction with what is shown in FIG. 2, the structures of the first secondary winding 143 and the second secondary winding 144 may be the same or different. When the first secondary winding 143 and the second secondary winding 144 are of the same structure, the leakage inductance L1 of the first transformer T1 and the leakage inductance L2 of the second transformer T2 under the initial configuration are equal, i.e., an initial leakage inductance difference is equal to zero. For example, the winding thickness of the first secondary winding 143 and the winding thickness of the second secondary winding 144 are equal and along the axial direction of the magnetic pillar 1410, and/or, the first overlap value N1between the first secondary winding 143 and the first primary winding 142 is equal to the second overlap value N2 between the second secondary winding 144 and the first primary winding 142. The first secondary winding 143 and the second secondary winding 144 are disposed symmetrically with respect to the center axis Q of the magnetic core 141. When the first secondary winding 143 and the second secondary winding 144 are not of the same structure, the leakage inductance L1 of the first transformer T1 and the leakage inductance L2 of the second transformer T2 under the initial configuration are not equal, i.e., the initial leakage inductance difference is not equal to zero. For example, the winding thickness of the first primary winding 142 is not equal to the winding thickness of the second secondary winding 144, and/or, the first overlap value N1 is not equal to the second overlap value N2 (e.g., as shown in FIG. 7 or FIG. 8, the first overlap value N1 between the first secondary winding 143 and the first primary winding 142 is less than the second overlap value N2 between the second secondary winding 144 and the first primary winding 142).

As another example, as shown in connection with FIG. 3, the first primary winding 142 is wound on an inner ring, the first secondary winding 143 and the second secondary winding 144 are wound on an outer ring and spaced apart along the radial direction (a direction perpendicular to the center axis O) of the magnetic pillar, and the first overlap value between the first primary winding 143 and the first primary winding 142 along the axial direction of the magnetic pillar, may be equal to or not equal to the second overlap value between the second secondary winding 144 and the first primary winding 142. When the first secondary winding 143 and the second secondary winding 144 are of the same structure, the leakage inductance L1 of the first transformer T1 and the leakage inductance L2 of the second transformer T2 under the initial configuration are equal, i.e., the initial leakage inductance difference is equal to zero. For example, the winding thickness of the first secondary winding 143 and the winding thickness of the second secondary winding 144 are equal and along the axial direction of the magnetic pillar, and/or, the first overlap value between the first secondary winding 143 and the first primary winding 142 is equal to the second overlap value between the second secondary winding 144 and the first primary winding 142. When the first secondary winding 143 and the second secondary winding 144 are not of the same structure, the leakage inductance L1 of the first transformer T1 and the leakage inductance L2 of the second transformer T2 under the initial configuration are not equal, and the initial leakage inductance difference is not equal to zero. For example, the winding thickness of the first primary winding 142 is not equal to the winding thickness of the second secondary winding 144, and/or, the first overlap value is not equal to the second overlap value (e.g., the first overlap value between the first secondary winding 143 and the first primary winding 142 is less than the second overlap value between the second secondary winding 144 and the first primary winding 142).

As another example, shown in conjunction with FIG. 4, the first primary winding 142 and the second primary winding 145 are wound on the inner ring, the first secondary winding 143 and the second secondary winding 144 are wound on the outer ring, and along the axial direction of the magnetic pillar (e.g., the first magnetic pillar 1411 or the second magnetic pillar 1412, with the first magnetic pillar and the second magnetic pillar being parallel), the first overlap value N1 between the first secondary winding 143 and the first primary winding 142 may be equal or unequal to, the second overlap value N2 between the second secondary winding 144 and the second primary winding 145, a turns ratio of the first primary winding 142 and the first secondary winding 143 may be equal to a turns ratio of the second primary winding 145 and the second secondary winding 144, a turns ratio of the first primary winding 142 and the second primary winding 145 may be equal or unequal to, a turns ratio of the first secondary winding 143 and the second secondary winding 144.

In some embodiments, the adjustment of at least one of the windings of the first transformer or the windings of the second transformer may be made at a factory design phase of the bipolar high-voltage generator or the transformer or during actual use after shipment.

In some embodiments, at the factory design phase, the first transformer T1 and the second transformer T2 are the same or near-identical structures, and an internal winding parameter, a position, and a material remain the same. Thus, the leakage inductance L1 of the first transformer T1 and the leakage inductance L2 of the second transformer T2 are equal, the leakage inductance difference is zero, and the first transformer T1 and the second transformer T2 are symmetrically disposed in the bipolar high-voltage generator 100. At this time, to ensure that the difference between the absolute value of the anode high voltage KV1 and the absolute value of the cathode high voltage KV2 falls within the preset range, the turns ratios, the positions, or the materials of the windings of at least one of the first transformer T1 or the second transformer T2 may be adjusted. The adjustment alters the leakage inductance L1 of the first transformer T1 and the leakage inductance L2 of the second transformer T2, thereby transforming the first transformer T1 and the second transformer T2 into an asymmetric state. At this time, the leakage inductance L1 of the first transformer T1 reaches a first target leakage inductance, and the leakage inductance L2 of the second transformer T2 reaches a second target leakage inductance, and the difference between the first target leakage inductance and the second target leakage inductance reaches the target leakage inductance difference. The first target leakage inductance divides the input voltage of the first transformer T1, and the second target leakage inductance divides the input voltage of the second transformer T2, so that the anode high-voltage output to the anode reaches the target anode high-voltage, and the cathode high-voltage output to the cathode reaches the target cathode high-voltage. The difference between the absolute value of the target anode high voltage and the absolute value of the target cathode high voltage reaches the target voltage difference (the target voltage difference is within the preset range), thereby further ensuring that the difference between the absolute value of the anode high voltage KV1 and the absolute value of the cathode high voltage KV2 falls within the preset range.

In some embodiments, due to factors such as component aging and temperature during the use of the bipolar high-voltage generator 100, the difference between the absolute value of the current anode high voltage KV1 and the absolute value of the current cathode high voltage KV2 changes, resulting in the leakage inductance L1 of the first transformer T1 and the leakage inductance L2 of the second transformer T2 being unequal, meaning the leakage inductance difference before the adjustment is not zero. At this time, to ensure that the difference between the absolute value of the anode high voltage KV1 and the absolute value of the cathode high voltage KV2 falls within the preset range or remains at the target voltage difference, the turns ratios, the positions, or the materials of the windings of at least one of the first transformer T1 or the second transformer T2 may be adjusted. The adjustment alters the leakage inductance L1 of the first transformer T1 and the leakage inductance L2 of the second transformer T2, thereby transforming the first transformer T1 and the second transformer T2 from a previous asymmetric state to another asymmetric state, and ultimately, ensuring that the leakage inductance L1 of the first transformer T1 reaches a third target leakage inductance and the leakage inductance L2 of the second transformer T2 reaches a fourth target leakage inductance. And at this time, a difference between the third target leakage inductance and the fourth target leakage inductance reaches the target leakage inductance difference corresponding to the adjusted target voltage difference, or remains at the original target leakage inductance difference. The third target leakage inductance divides the input voltage of the first transformer T1, and the fourth target leakage inductance divides the input voltage of the second transformer T2, so that the anode high-voltage output to the anode reaches the target anode high-voltage, and the cathode high-voltage output to the cathode reaches the target cathode high-voltage. Thus, the difference between the absolute value of the target anode high-voltage and the absolute value of the target cathode high-voltage reaches the target voltage difference (the target voltage difference is within the preset range), further achieving the purpose of balancing the output voltage and delivering the desired target voltage difference.

The target leakage inductance of the first transformer T1 being greater than the target leakage inductance of the second transformer T2 may be achieved through at least one of the following operations, including: maintaining the leakage inductance of the first transformer T1 unchanged while reducing the leakage inductance of the second transformer T2; or, maintaining the leakage inductance of the second transformer T2 unchanged while increasing the leakage inductance of the first transformer T1; or, simultaneously adjusting the leakage inductances of both the first transformer T1 and the second transformer T2, with an justment increment of the leakage inductance of the first transformer T1 being greater than an justment increment of the leakage inductance of the second transformer T2; or, simultaneously adjusting the leakage inductances of both the first transformer T1 and the second transformer T2, with an adjustment decrement of the leakage inductance of the first transformer T1 being smaller than an adjustment decrement of the leakage inductance of the second transformer T2.

Similarly, the target leakage inductance of the first transformer T1 being less than the target leakage inductance of the second transformer T2 may be achieved through at least one of the following operations, including: maintaining the leakage inductance of the first transformer T1 unchanged while increasing the leakage inductance of the second transformer T2; or, maintaining the leakage inductance of the second transformer T2 unchanged while decreasing the leakage inductance of the first transformer T1; or, simultaneously adjusting the leakage inductances of both the first transformer T1 and the second transformer T2, with the adjustment increment of the leakage inductance of the first transformer T1 being smaller than the adjustment increment of the leakage inductance of the second transformer T2; or, simultaneously adjusting the leakage inductances of both the first transformer T1 and the second transformer T2, with the adjustment decrement of the leakage inductance of the first transformer T1 being greater than the adjustment decrement of the leakage inductance of the second transformer T2.

For example, before the adjustment, the leakage inductance L1 of the first transformer T1 is 10 µH, and the leakage inductance L2 of the second transformer T2 is 5 µH, corresponding to an initial leakage inductance difference of 5 µH. Assuming that before the adjustment, the anode high-voltage KV1 is 60 kV and the cathode high-voltage KV2 is -50 kV, and the difference between the absolute value of the anode high-voltage KV1 and the absolute value of the cathode high-voltage KV2 is 10 kV. Assuming that the target anode high-voltage is 55 kV, the target cathode high-voltage is -50 kV, and the target voltage difference is 5 kV. At this time, the leakage inductance L1 of the first transformer T1 may be increased to 15 µH, while the leakage inductance L2 of the second transformer T2 is maintained at 5 µH, ensuring that the leakage inductance difference after the adjustment reaches the target leakage inductance difference of 10 µH. The adjustment increases the voltage division of the leakage inductance L1 of the first transformer T1, thereby reducing the anode high voltage KV1 from 60 kV to the target anode high voltage of 55 kV, transforming the difference between the absolute value of the anode high voltage KV1 and the absolute value of the cathode high voltage KV2 to 5 kV, achieving the target voltage difference.

As another example, before the adjustment, the leakage inductance L1 of the first transformer T1 is 10 µH, and the leakage inductance L2 of the second transformer T2 is 5 µH, corresponding to the initial leakage inductance difference of 5 µH. Assuming that before the adjustment, the anode high-voltage KV1 is 60 kV, the cathode high-voltage KV2 is -50 kV, and the voltage difference between the absolute value of the anode high-voltage KV1 and the absolute value of the cathode high-voltage KV2 is 10 kV. Assuming that the target anode high-voltage is 55kV, the target cathode high-voltage is -50 kV, and the target voltage difference is 5 kV. At this time, the leakage inductance L1 of the first transformer T1 may be increased to 20 µH, and the leakage inductance L2 of the second transformer T2 may be increased to 10 µH, maintaining the leakage inductance difference after the adjustment at 10 µH. The adjustment reduces the anode high voltage KV1 from 60 kV to the target anode high voltage of 55 kV, and transforms the difference between the absolute value of the anode high voltage KV1 and the absolute value of the cathode high voltage KV2 to 5 kV, achieving the target voltage difference.

In some embodiments, the adjustment module 530 may perform at least one of the following adjustment operations to make the bipolar high-voltage generator produce the target leakage inductance difference: adjusting the first overlap value based on the first adjustment parameter, adjusting the second overlap value based on the second adjustment parameter, adjusting the winding thickness of the secondary winding of the first transformer (e.g., the first secondary winding) based on the first adjustment parameter, and adjusting the winding thickness of the secondary winding of the second transformer (e.g., the second secondary winding) based on the second adjustment parameter. For example, when adjusting the leakage inductance L1 of the first transformer T1, the first overlap value N1 of the first transformer T1 or the winding thickness of the first secondary winding 143 may be adjusted; accordingly, when adjusting the leakage inductance L2 of the second transformer T2, the second overlap value N2 of the second transformer T2 or the winding thickness of the first secondary winding 143 may be adjusted, thereby adjusting the target leakage inductance difference corresponding to the output and the target voltage difference corresponding to the target leakage inductance difference.

In some embodiments, based on the structures of the first transformer T1 and the second transformer T2, to realize the output voltage difference within the preset range (e.g., to achieve the target voltage difference) and thereby meet voltage balancing or different power supply requirements, when adjusting the leakage inductance of the first transformer T1, the first overlap value N1 of the first transformer T1 or the winding thickness of the first secondary winding 143 may be adjusted. Correspondingly, when adjusting the leakage inductance of the second transformer T2, the second overlap value N2 of the second transformer T2 or the winding thickness of the second secondary winding 144 may be adjusted, to adjust the target leakage inductance difference corresponding to the output and the target voltage difference corresponding to the target leakage inductance difference. When only one of the overlap value and the winding thickness is adjusted, the other parameter is fixed.

In some embodiments, the adjustment module 530 may determine to perform a target adjustment operation based on the determined adjustment mode. For example, if the adjustment mode is determined to adjust the first transformer, the adjustment module 530 may perform the target adjustment operation: adjusting the first overlap value based on the first adjustment parameter, and adjusting the winding thickness of the first secondary winding based on the first adjustment parameter to make the bipolar high-voltage generator produce the target leakage inductance difference. For example, if it is determined that the adjustment mode is to adjust the first transformer and the second transformer, the adjustment module 530 may perform the target regulation operation: adjusting the first overlap value based on the first adjustment parameter, adjusting the second overlap value based on the second adjustment parameter, adjusting the winding thickness of the first secondary winding based on the first adjustment parameter, and adjusting the winding thickness of the second secondary winding based on the second adjustment parameter.

In some embodiments, the adjustment module 530 may determine to perform the target adjustment operation based on the determined adjustment mode and the parameter type. For example, if it is determined that the adjustment mode is to adjust the second transformer, and the parameter type to be adjusted is the overlap value, the adjustment module 530 may perform the target regulating operation: adjusting the second overlap value of the second transformer based on the second adjustment parameter to make the bipolar high-voltage generator produce the target leakage inductance difference. For example, if it is determined that the adjustment mode is to adjust the first transformer, and the parameter type to be adjusted is the overlap value and the winding thickness, the adjustment module 530 may perform the target adjustment operation: adjusting the first overlap value of the first transformer based on the first adjustment parameter, and adjusting the winding thickness of the first secondary winding of the first transformer based on the first adjustment parameter, to make the bipolar high-voltage generator produce the target leakage inductance difference. As another example, if it is determined that the adjustment mode is to adjust both the first transformer and the second transformer, and the parameter type to be adjusted is the overlap value (or the winding thickness), the adjustment module 530 may perform the target adjustment operation: adjusting the first overlap value of the first transformer based on the first adjustment parameter, and adjusting the second overlap value of the second transformer based on the second adjustment parameter (or adjusting the winding thickness of the first secondary winding of the first transformer based on the first adjustment parameter, and adjusting the winding thickness of the second secondary winding of the second transformer based on the second adjustment parameter) to make the bipolar high-voltage generator produce the target leakage inductance difference.

In some embodiments, the control system 500 may perform at least one of the following operations to make an overlap difference (also referred to as a target overlap difference) between the first overlap value and the second overlap value correspond to the target leakage inductance difference, and the overlap difference is not zero: adjusting the first overlap value based on the first adjustment parameter, and adjusting the second overlap value based on the second adjustment parameter. That is, to output the target voltage difference, the first overlap value of the first transformer T1 and the second overlap value of the second transformer T2 may be adjusted relative to each other, to make the difference between the first overlap value and the second overlap value reach the target overlap difference corresponding to the target leakage inductance difference, which in turn makes the difference between the leakage inductance of the first transformer T1 and the leakage inductance of the second transformer T2 reach the target leakage inductance difference, thereby adjusting the target voltage difference.

Exemplarily, if the first overlap value N1 is equal to the second overlap value N2 in the initial configuration, the winding thickness of the first secondary winding of the first transformer T1 is equal to the winding thickness of the second secondary winding of the second transformer T2, that is, the leakage inductance L1 of the first transformer T1 is equal to the leakage inductance L2 of the second transformer T2. To realize the balance of the output voltage or ensure that the target voltage difference is less than zero, when the winding thickness of the first secondary winding is equal to the winding thickness of the second secondary winding, it is possible to adjust the first overlap value N1 to be less than the second overlap value N2, and ensure that the difference between the both reaches the target overlap difference. The balance of the output voltage means that the absolute value of the target cathode high-voltage of the final output is equal to the absolute value of the target anode high-voltage, and the target voltage difference being less than zero means that the absolute value of the target cathode voltage of the final output is greater than the absolute value of the target anode voltage. The aforementioned adjustment may change at least one of the leakage inductance L1 of the first transformer T1 or the leakage inductance L2 of the second transformer T2, ensuring that the leakage inductance L1 of the first transformer T1 is greater than the leakage inductance L2 of the second transformer T2, and the difference between the leakage inductance L1 and the leakage inductance L2 reaches the target leakage inductance difference. After the adjustment, the target leakage inductance of the first transformer T1 performs a voltage division process on the output circuit of the anode high-voltage KV1. A voltage division value of the target leakage inductance of the first transformer T1 is greater than a voltage division value of the target leakage inductance of the second transformer T2, thereby ensuring that the absolute value of the final target anode high-voltage output to the anode 11 is close to or equal to the absolute value of the target cathode high-voltage output to the cathode 12, which achieves the goal of balancing the output voltage or ensuring that the output target voltage difference is less than zero.

In some embodiments, when the winding thickness of the first secondary winding is equal to the winding thickness of the second secondary winding, the first overlap value N1 being less than the second overlap value N2 may be realized through at least one of the following operations: remaining the first overlap value N1 unchanged and increasing the second overlap value N2; or, remaining the second overlap value N2 unchanged and decreasing the first overlap value N1; or, simultaneously adjusting the first overlap value N1 and the second overlap value N2, with an adjustment decrement of the first overlap value N1 is greater than an adjustment decrement of the second overlap value N2; or, simultaneously adjusting the first overlap value N1 and the second overlap value N2, with an adjustment increment of the first overlap value N1 is smaller than an adjustment increment of the second overlap value N2. The foregoing operation may make a trend of the change in the overlap difference between the first overlap value N1 and the second overlap value N2 exhibit a correlation with a trend of the change in the voltage difference between the absolute value of the initial anode high-voltage KV1 and the absolute value of the initial cathode high-voltage KV2.

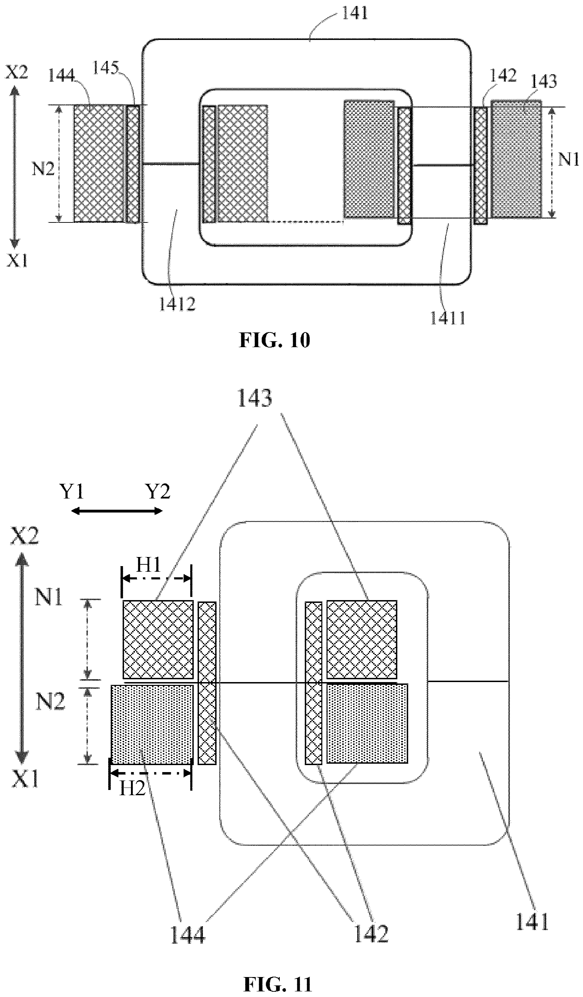

FIG. 7-FIG. 10 are schematic diagrams illustrating a process for adjusting overlap values of transformers according to some embodiments of the present disclosure. FIG. 7-FIG. 9 are schematic diagrams illustrating winding adjustment corresponding to a first structure of a transformer illustrated in FIG. 2, and FIG. 10 is a schematic diagram illustrating winding adjustment corresponding to a third structure of a transformer illustrated in FIG. 4.

When the winding thicknesses of the secondary windings of the first transformer and the second transformer are equal, to realize that the first overlap value N1 is smaller than the second overlap value N2, as shown in FIG. 7, the first secondary winding 143 and the second secondary winding 144 may be simultaneously moved along a second direction X2. At this time, the first overlap value N1 decreases, while the second overlap value N2 remains unchanged. As a result, the first overlap value N1 becomes smaller than the second overlap value N2, and the overlap difference between the first overlap value N1 and the second overlap value N2 corresponds to the target leakage inductance difference. The leakage inductance L1 of the first transformer T1 increases, and the leakage inductance L2 of the second transformer T2 remains unchanged, ensuring that the voltage division of the leakage inductance L1 of the first transformer T1 is greater than the voltage division of the leakage inductance L2 of the second transformer T2.

Alternatively, the position of the second secondary winding 144 remains unchanged, the first secondary winding 143 is moved in the second direction X2, as shown in FIG. 8 and FIG. 10. At this time, the first overlap value N1 becomes smaller and the second overlap value N2 remains unchanged, thus, the first overlap value N1 is smaller than the second overlap value N2, and the overlap difference between the first overlap value N1 and the second overlap value N2 corresponds to the target leakage inductance difference. The leakage inductance L1 of the first transformer T1 increases, and the leakage inductance L2 of the second transformer T2 remains unchanged, resulting in that the voltage division of the leakage inductance L1 of the first transformer T1 is greater than the voltage division of the leakage inductance L2 of the second transformer T2.

Alternatively, as shown in FIG. 9, the first secondary winding 143 is moved along the second direction X2 and the second secondary winding 144 is moved along a first direction X1, and an offset amount of the first secondary winding 143 is greater than an offset amount of the second secondary winding 144. As a result, the first overlap value N1 is less than the second overlap value N2, and the overlap difference between the first overlap value N1 and the second overlap value N2 corresponds to the target leakage inductance difference. The leakage inductance L1 of the first transformer T1 and the leakage inductance L2 of the second transformer T2 both increase, but the adjustment increment of the leakage inductance L1 of the first transformer T1 is greater than the adjustment increment of the leakage inductance L2 of the second transformer T2, ensuring that the voltage division of the first Leakage inductance L1 of the first transformer T1 is greater than the voltage division of the leakage inductance L2 of the second transformer T2.

In conjunction with the above example, when the target voltage difference to be output is greater than zero, i.e., the absolute value of the target anode high-voltage to be ultimately output is greater than the absolute value of the target cathode high-voltage, and the target leakage inductance of the first transformer T1 needs to be smaller than the target leakage inductance of the second transformer T2. When the winding thicknesses of the first secondary winding and the second secondary winding are equal, the first overlap value N1may be adjusted to be greater than the second overlap value N2. The adjustment changes at least one of the leakage inductance L1 of the first transformer T1 or the leakage inductance L2 of the second transformer T2, making that the leakage inductance L1 of the first transformer T1 is smaller than the leakage inductance L2 of the second transformer T2. After the adjustment, the target leakage inductance of the first transformer T1 performs the voltage division process on the output circuit of the anode high-voltage KV1. The voltage division of the target leakage inductance of the first transformer T1 is smaller than the voltage division of the target leakage inductance of the second transformer T2. As a result, the absolute value of the target anode high-voltage ultimately output to the anode 11 is greater than the absolute value of the target cathode high-voltage, achieving the purpose of the target voltage difference greater than zero.

In some embodiments, when the winding thicknesses of the first secondary winding and the second secondary winding are equal, the first overlap value N1 being greater than the second overlap value N2 may be achieved through at least one of the following operations: maintaining the first overlap value N1 unchanged while decreasing the second overlap value N2; or maintaining the second overlap value N2 unchanged while increasing the first overlap value N1; or simultaneously adjusting both the first overlap value N1 and the second overlap value N2, with the adjustment increment of the first overlap value N1 being greater than the adjustment increment of the second overlap value N2. The foregoing operation may make a trend of the change in the overlap difference between the first overlap value N1 and the second overlap value N2 exhibit a correlation with a trend of the change in the voltage difference between the absolute value of the initial anode high-voltage KV1 and the absolute value of the initial cathode high-voltage KV2.

When the winding thicknesses of the secondary windings of the first transformer and the second transformer are equal, in conjunction with what is shown in FIG. 2 or FIG. 4, to realize that the first overlap value N1 is greater than the second overlap value N2, the first secondary winding 143 and the second secondary winding 144 may be simultaneously moved along the first direction X1. At this time, the first overlap value N1 remains unchanged, and the second overlap value N2 decreases; therefore, the first overlap value N1 is greater than the second overlap value N2. The leakage inductance L1 of the first transformer T1 remains unchanged, the leakage inductance L2 of the second transformer T2 increases, and the voltage division of the leakage inductance L2 of the second transformer T2 is greater than the voltage division of the leakage inductance L1 of the first transformer T1. Alternatively, the position of the first secondary winding 143 remains unchanged, and the second secondary winding 144 is moved along the first direction X1. At this time, the first overlap value N1 remains unchanged, and the second overlap value N2 becomes smaller, thus the first overlap value N1 is greater than the second overlap value N2. The leakage inductance L1 of the first transformer T1 remains unchanged, the leakage inductance L2 of the second transformer T2 increases, and the voltage division of the leakage inductance L2 of the second transformer T2 is greater than the voltage division of the leakage inductance L1 of the first transformer T1. Alternatively, the second secondary winding 144 is moved along the first direction X1, and the first secondary winding 143 is moved along the second direction X2, and the offset amount of the second secondary winding 144 is greater than the offset amount of the first secondary winding 143. As a result, the second overlap value N2 is smaller than the first overlap value N1, and the leakage inductance L1 of the first transformer T1 and the leakage inductance L2 of the second transformer T2 both increase. The adjustment increment of the leakage inductance L2 of the second transformer T2 is larger than the adjustment increment of the leakage inductance L2 of the first transformer T1, and the voltage division of the leakage inductance L2 of the second transformer T2 is greater than the voltage division of the leakage inductance L1 of the first transformer T1.

In some embodiments, the control system 500 may perform at least one of the following operations to make a thickness difference (also referred to as a target thickness difference) between the winding thickness of the secondary winding of the first transformer (e.g., the first secondary winding) and the winding thickness of the secondary winding of the second transformer (e.g., the second secondary winding) correspond to the target leakage inductance difference, and the thickness difference is not zero: adjusting the winding thickness of the secondary winding of the first transformer based on the first adjustment parameter, and adjusting the winding thickness of the secondary winding of the second transformer based on the second adjustment parameter. By relatively adjusting the winding thickness of the first secondary winding of the first transformer T1 and the winding thickness of the second secondary winding of the second transformer T2, different winding thicknesses may be achieved, ensuring that the difference between the leakage inductance of the first transformer T1 and the leakage inductance of the second transformer T2 reaches the target leakage inductance difference, thereby adjusting the output of the target voltage difference.

Exemplarily, if the first overlap value N1 is equal to the second overlap value N2 in the initial configuration, the winding thickness of the first secondary winding of the first transformer T1 is equal to the winding thickness of the second secondary winding of the second transformer T2, i.e., the leakage inductance L1 of the first transformer T1 is equal to the leakage inductance L2 of the second transformer T2. To realize the balancing of the output voltage or to realize the target voltage difference of less than zero, when the first overlap value N1 is equal to the second overlap value N2, the winding thickness of the first secondary winding of the first transformer T1 and the winding thickness of the second secondary winding of the second transformer T2 may be adjusted to make that the winding thickness of the first secondary winding is greater than the winding thickness of the second secondary winding. The foregoing adjustment may change at least one of the leakage inductance L1 of the first transformer T1 or the leakage inductance L2 of the second transformer T2 to make that the leakage inductance L1 of the first transformer T1 is greater than the leakage inductance L2 of the second transformer T2. After the adjustment, the leakage inductance L1 of the first transformer T1 performs the voltage division process on the output circuit of the anode high voltage KV1. The voltage division of the leakage inductance L1 of the first transformer T1 is greater than the voltage division of the leakage inductance L2 of the second transformer T2. As a result, the absolute value of the target anode high-voltage ultimately output to the anode 11 is close to or equal to the absolute value of the target cathode high-voltage, achieving the goal of balancing the output voltage or ensuring that the target voltage difference is less than zero.

When the first overlap value N1 is equal to the second overlap value N2, with reference to FIG. 2 or FIG. 4, the winding thickness of the first secondary winding 143 being greater than the winding thickness of the second secondary winding 144 may be realized by at least one of the following operations, including: containing the winding thickness of the first secondary winding 143 unchanged, and decreasing the winding thickness of the second secondary winding 144; or, containing the winding thickness of the second secondary winding 144 unchanged, and increasing the winding thickness of the first secondary winding 143; or, simultaneously increasing the winding thickness of the first secondary winding 143 and the winding thickness of the second secondary winding 144, with an adjustment increment of the winding thickness of the first secondary winding 143 is greater than an adjustment increment of the winding thickness of the second secondary winding 144; or, simultaneously decreasing the winding thickness of the first secondary winding 143 and the winding thickness of the second secondary winding 144, with an adjustment decrement of the winding thickness of the first secondary winding 143 is less than an adjustment decrement of the winding thickness of the second secondary winding 144.

When the first overlap value N1 is equal to the second overlap value N2, and the winding thickness of the first secondary winding 143 is greater than the winding thickness of the second secondary winding 144, a first distance of the first secondary winding 143 from the corresponding primary winding (e.g., the first primary winding 142) is greater than a second distance of the second secondary winding 144 from the corresponding primary winding (e.g., the first primary winding 142 or the second primary winding 145). As a result, the leakage inductance of the first transformer T1 is greater than the leakage inductance of the second transformer T2, and the voltage division of the leakage inductance L1 of the first transformer T1 is greater than the voltage division of the leakage inductance L2 of the second transformer T2.

FIG. 11 and FIG. 12 are schematic diagrams illustrating a process for adjusting a winding thickness of a transformer according to some embodiments of the present disclosure. FIG. 11 is a schematic diagram illustrating winding adjustment corresponding to a first structure of a transformer shown in FIG. 2, and FIG. 12 is a schematic diagram illustrating winding adjustment corresponding to a third structure of a transformer shown in FIG. 4.

Continuing with the above example, when the target voltage difference to be output is greater than zero, the leakage inductance L1 of the first transformer T1 is required to be smaller than the leakage inductance L2 of the second transformer T2, and when the first overlap value and the second overlap value are equal, as shown in FIG. 11 or FIG. 12, the winding thickness H1 of the first secondary winding 143 may be adjusted to be smaller than the winding thickness H2 of the second secondary winding 144. The adjustment may change at least one of the leakage inductance L1 of the first transformer T1 or the leakage inductance L2 of the second transformer T2, so that the leakage inductance L1 of the first transformer T1 is less than the leakage inductance L2 of the second transformer T2. After the adjustment, the leakage inductance L1 of the first transformer T1 performs the voltage division process on the output circuit of the anode high-voltage KV1. The voltage division of the leakage inductance L1 of the first transformer T1 is smaller than the voltage division of the leakage inductance L2 of the second transformer T2. As a result, the absolute value of the target anode high-voltage ultimately output to the anode 11 is greater than the absolute value of the target cathode high-voltage output to the cathode 12, achieving the goal of the target voltage difference greater than zero.

Similarly, when the first overlap value N1 is equal to the second overlap value N2, as shown in FIG. 2 or FIG. 4, the winding thickness H1 of the first secondary winding 143 being less than the winding thickness H2 of the second secondary winding 144 may be achieved through at least one of the following operations: maintaining the winding thickness H1 of the first secondary winding 143 unchanged while increasing the winding thickness H2 of the second secondary winding 144; or, maintaining the winding thickness H2 of the second secondary winding 144 unchanged while decreasing the winding thickness H1 of the first secondary winding 143; or, simultaneously increasing the winding thickness H1 of the first secondary winding 143 and the winding thickness H2 of the second secondary winding 144, with the adjustment increment of the winding thickness H1 of the first secondary winding 143 being smaller than the adjustment increment of the winding thickness H2 of the second secondary winding 144; or, simultaneously decreasing the winding thickness H1 of the first secondary winding 143 and the winding thickness H2 of the second secondary winding 144, with the adjustment decrement of the winding thickness H1 of the first secondary winding 143 being greater than the adjustment decrement of the winding thickness H2 of the second secondary winding 144.

In some embodiments, the adjustment of the winding thickness may be accomplished by adjusting a count of turns of the winding while maintaining a fixed transformer ratio, increasing or decreasing a diameter of the winding, or the like. For example, the winding thickness H1 of the first secondary winding 143 may be increased by increasing the count of turns of the first secondary winding 143 wound onto the first primary winding 142 while maintaining a fixed transformer ratio of the first transformer T1.

It should be noted that, considering that the secondary winding of the transformer is wound on the primary winding, a gap between the primary winding and the secondary winding is zero or close to zero in practice, resulting in less room for adjustability of the winding thickness of the primary winding. Therefore, in the aforementioned embodiments, it is exemplified that the adjustment parameter may include the winding thickness of the secondary windings (e.g., the first adjustment parameter includes the winding thickness of the first secondary winding 143, and the second adjustment parameter includes the winding thickness of the second secondary winding 144). Under permissible conditions, such as when there is a certain gap between the secondary winding and the primary winding, it is possible to adjust the winding thickness of the primary winding. Thus, the adjustment parameter may also include the winding thickness of the primary winding, meaning that the winding thickness of the primary winding of at least one of the first transformer or the second transformer may be adjusted.

In some embodiments of the present disclosure, after obtaining the target voltage difference within the preset range, the windings of at least one of the first transformer T1 or the second transformer T2 of the high-voltage generator 100 are adjusted based on the target voltage difference to be output, which changes the difference between the leakage inductance L1 of the first transformer T1 and the leakage inductance L2 of the second transformer T2 to achieve the target leakage inductance difference. After the adjustment, the leakage inductance L1 of the first transformer T1 and the leakage inductance L2 of the second transformer T2 correspond to the voltage division in the output circuit of the anode high-voltage KV1 and the cathode high-voltage KV2, respectively, which ensures that the difference between the absolute value of the target anode high-voltage and the absolute value of the target cathode high-voltage output by the bipolar high-voltage generator reaches the target voltage difference, thereby containing the difference between the absolute value of the anode high-voltage KV1 and the absolute value of the cathode high-voltage KV2 within the preset range. When the goal is to achieve the balancing of the output voltage, the target voltage difference can be set to zero or close to zero. By adjusting the windings of at least one of the first transformer T1 or the second transformer T2, the output voltage difference can reach the target voltage difference, achieving the purpose of balancing the output voltage.

In some embodiments, the adjustment module 530 adjusts, by an inverter circuit, the working frequency of at least one of the first transformer and the second transformer, based on the target voltage difference or the adjustment parameter, to make the absolute value of the anode voltage output from the bipolar high-voltage generator and the absolute value of the cathode voltage output from the bipolar high-voltage generator fall within the preset range.

In some embodiments, the adjustment module 530 adjusts the working frequency of the first transformer by the inverter circuit to make the difference between the absolute value of the anode voltage output by the bipolar high-voltage generator and the absolute value of the cathode voltage output by the bipolar high-voltage generator fall within the preset range. In some embodiments, the adjustment module 530 adjusts the working frequency of the second transformer by the inverter circuit to make the difference between the absolute value of the anode voltage output by the bipolar high-voltage generator and the absolute value of the cathode voltage output by the bipolar high-voltage generator fall within the preset range.

Because the first transformer and the second transformer are powered through the same inverter circuit, adjusting the working frequency of one of the transformers changes the other.

In some embodiments, the adjustment module 530 adjusts the working frequency of at least one of the first transformer and the second transformer by the inverter circuit to increase the gain of the cathode voltage output from the bipolar high-voltage generator, and decrease the gain of the anode voltage, thereby making the difference between the absolute value of the target anode voltage and the absolute value of the target cathode voltage output by the bipolar high-voltage generator satisfy the target voltage difference.

More details regarding the adjustment of the working frequency may be found in FIG. 14 and its related descriptions.

In some embodiments, the adjustment of the working frequency of at least one of the first transformer and the second transformer, and the adjustment of the leakage inductance, may be performed independently of each other, or sequentially. For example, after obtaining the target voltage difference, the adjustment module 530 may adjust the working frequency of at least one of the first transformer or the second transformer directly through the inverter circuit to make the difference between the absolute value of the target anode voltage output by the bipolar high-voltage generator and the absolute value of the target cathode voltage output by the bipolar high-voltage generator satisfy the target voltage difference. As another example, after obtaining the target voltage difference, the adjustment module 530 may adjust the windings of at least one of the first transformer or the second transformer to make the bipolar high-voltage generator output the target leakage inductance difference, thereby ensuring that the difference between the absolute value of the target anode voltage output by the bipolar high-voltage generator and the absolute value of the target cathode voltage output by the bipolar high-voltage generator satisfies the target voltage difference. As yet another example, after adjusting the windings of at least one of the first transformer or the second transformer to make the bipolar high-voltage generator output the target leakage inductance difference, the adjustment module 530 may obtain the current cathode high-voltage and the current anode high-voltage of the bipolar high-voltage generator, and determine whether the difference between the absolute value of the anode high-voltage and the absolute value of the cathode high-voltage falls within the preset range. In response to determining that the difference is not within the preset range, the adjustment module 530 may update the target voltage difference based on the difference and the preset range, and further adjust the working frequency of at least one of the first transformer or the second transformer through the inverter circuit based on the updated target voltage difference to make the difference between the absolute value of the anode voltage output by the bipolar high-voltage generator and the absolute value of the cathode voltage output by the bipolar high-voltage generator fall within the preset range.

In some embodiments of the present disclosure, the method does not require the addition of extra inverter circuit structures, thereby simplifying the design of the high-voltage generator 100 and the X-ray system, reducing design costs, and enhancing system reliability. Additionally, it allows for corresponding adjustments based on the output voltage requirements, meeting the needs of different working scenarios.

FIG. 13 is an exemplary flowchart illustrating a process for determining an adjustment parameter according to some embodiments of the present disclosure. As shown in FIG. 13, process 1300 includes the following operations. In some embodiments, the process 1300 may be performed by the control system 500 (e.g., the parameter determination module 520).

In 1310, a reference leakage inductance difference between a first transformer and a second transformer may be determined based on a target voltage difference.

The reference leakage inductance difference refers to the difference between a reference leakage inductance of the first transformer and a reference leakage inductance of the second transformer.

In some embodiments, the reference leakage inductance difference may be determined by mathematical computation, simulation, or a trained leakage inductance difference determination model.

Exemplarily, after obtaining the target voltage difference, the control system 500 may determine the reference leakage inductance of the first transformer T1 and the reference leakage inductance of the second transformer T2 through simulation or mathematical computation based on a current circuit parameter and a circuit structure of the bipolar high-voltage generator 100, and then determine the reference leakage inductance difference between the first transformer T1 and the second transformer T2. In this embodiment, since the reference leakage inductance difference is not an actual value but rather a predicted value obtained through simulation or computation, the reference leakage inductance difference is also referred to as a theoretical leakage inductance difference. Correspondingly, the reference leakage inductance is also referred to as a theoretical leakage inductance.

Further exemplarily, after obtaining the target voltage difference, the control system 500 may input a circuit model of the bipolar high-voltage generator (including the circuit structure and the circuit parameter) as input data, or input both the target voltage difference and the circuit model of the bipolar high-voltage generator as the input data, into a trained leakage inductance difference determination model. The leakage inductance difference determination model, after analysis and processing, outputs the corresponding reference leakage inductance difference, and/or, the reference leakage inductance of the first transformer and the reference leakage inductance of the second transformer.

In some embodiments, the leakage inductance difference determination model may be a different model from a parameter determination model. For example, the leakage inductance difference determination model may be a model obtained by training an initial machine learning model using a training sample (also referred to as a second training sample). Exemplarily, the second training sample may include at least one of different sample target voltage differences or reference leakage inductance differences corresponding to different sample circuit models (the circuit models of the bipolar high-voltage generator).

In some embodiments, the leakage inductance difference determination model may be part of the parameter determination model. For example, if the parameter determination model includes an embedding layer, an encoding layer, an intermediate layer, a decoding layer, and a fully connected layer that are sequentially connected, the leakage inductance difference determination model may be the portion including the embedding layer, the encoding layer, and the intermediate layer. After inputting the target voltage difference and the circuit model of the bipolar high-voltage generator into the parameter determination model, the intermediate layer of the parameter determination model may output the reference leakage inductance difference.

In 1320, an adjustment parameter may be determined based on the reference leakage inductance difference.

In conjunction with the above, the adjustment parameter may include at least one of a first adjustment parameter or a second adjustment parameter.

In some embodiments, at least one of the first adjustment parameter or the second adjustment parameter may be determined based on the reference leakage inductance difference by mathematical computation, simulation, or a trained machine learning model (e.g., the leakage inductance difference determination model and the parameter determination model).

Exemplarily, after determining the reference leakage inductance of the first transformer T1, the reference leakage inductance of the second transformer T2, and the reference leakage inductance difference between the first transformer T1 and the second transformer T2, the control system 500 may read a current anode high-voltage and a current cathode high-voltage output by the bipolar high-voltage generator, and based on the current anode high-voltage and the current cathode high-voltage, determine a voltage adjustment range (e.g., a range within which the anode high-voltage needs to be adjusted, a range within which the cathode high-voltage needs to be adjusted, or a range within which the voltage difference needs to be adjusted). Subsequently, the control system 500 may determine at least one of an adjustment amount of the first adjustment parameter for a winding of the first transformer (e.g., an adjustment amount of a first overlap value, or an adjustment amount of a winding thickness of a first secondary winding) or the second adjustment parameter for a winding of the second transformer (e.g., an adjustment amount of a second overlap value, or an adjustment amount of a winding thickness of a second secondary winding) through simulation or mathematical computation based on a structure model of the transformer.

Further exemplarily, the target voltage difference, the current anode high-voltage, and the current cathode high-voltage output from the bipolar high-voltage generator, and the circuit model of the bipolar high-voltage generator are used as the input data, or the target voltage difference and the circuit model of the bipolar high-voltage generator are used as the input data. After the control system 500 inputs the input data into the trained leakage inductance difference determination model or the parameter determination model, the leakage inductance difference determination model or the parameter determination model analyzes and processes the data to determine the reference leakage inductance of the first transformer T1 and the reference leakage inductance of the second transformer T2, and the reference leakage inductance difference between the first transformer T1 and the second transformer T2, further analyzes and processes to determine the adjustment amount of at least one of the first adjustment parameter or the second adjustment parameter, which is then output.

Based on this, when it is necessary to output the target voltage difference, the control system 500 may determine the reference leakage inductance of the first transformer T1, the reference leakage inductance of the second transformer T2, and the reference leakage inductance difference based on the target voltage difference and the circuit model of the bipolar high-voltage generator, subsequently, determine the first overlap value and the second overlap value (or an overlap difference between the first overlap value and the second overlap value) required to achieve the target voltage difference, and/or, winding thicknesses required to be achieved (or a winding thickness difference) for the first transformer and the second transformer, and based on the determined values, adjust at least one of the overlap value or the winding thickness of at least one of the first transformer or the second transformer.

In 1330, a leakage inductance of at least one of the first transformer and the second transformer may be adjusted based on the adjustment parameter.

In some embodiments, the adjustment module 530 may adjust at least one of the first overlap value of the windings of the first transformer, or the winding thickness of the windings of the first transformer based on the first adjustment parameter. In some embodiments, the adjustment module 530 may adjust at least one of the second overlap value of the windings of the second transformer, or the winding thickness of the windings of the second transformer based on the second adjustment parameter. In some embodiments, the adjustment module 530 may adjust at least one of the first overlap value of the windings of the first transformer, or the winding thickness of the windings of the first transformer based on the first adjustment parameter; and adjust at least one of the second overlap value of the windings of the second transformer, or the winding thickness of the windings of the second transformer based on the second adjustment parameter.

For example, after obtaining the target voltage difference, the control system 500 may determine the reference leakage inductance of the first transformer T1, the reference leakage inductance of the second transformer T2, and the reference leakage inductance difference by combining the circuit model of the bipolar high-voltage generator 100 and performing circuit simulation (e.g., using a simulation software such as Multisim, Ansys, etc.) to construct a circuit module with corresponding parameters. The control system 500 may read an initial anode high-voltage KV1 and an initial cathode high-voltage KV2 at a current time, determine the voltage adjustment range based on the initial anode high-voltage and the initial cathode high-voltage, and, in conjunction with the structure model of the transformer, determine required parameter adjustments (e.g., an overlap adjustment value, a winding thickness adjustment value) for the leakage inductance of the first transformer T1 and the second transformer T2. By manually or using an adjustment device to adjust at least one of the first transformer T1 or the second transformer T2 according to the adjustment parameter, the target leakage inductance of the first transformer T1, the target leakage inductance of the second transformer T2, and the target leakage inductance difference may be practically achieved, which ensures that the anode high-voltage KV1 output to the anode 11 reaches the target anode high-voltage, the cathode high-voltage KV2 output to the cathode 12 reaches the target cathode high-voltage, and the difference between the absolute value of the target anode high-voltage and the absolute value of the target cathode high-voltage satisfies the required target voltage difference.

More details regarding the adjustment of the leakage inductance may be found in FIG. 6 and its related descriptions, which will not be repeated here.

FIG. 14 is an exemplary flowchart illustrating a method of controlling a bipolar high-voltage generator according to some other embodiments of the present disclosure. In some embodiments, process 1400 may be performed by the control system 500 or a control device described in FIG. 1.

In 1410, a target voltage difference of a bipolar high-voltage generator may be obtained, wherein the target voltage difference is within a preset range.

In some embodiments, the control device may obtain the target voltage difference in real time while the bipolar high-voltage generator (e.g., the bipolar high-voltage generator 100) is working (e.g., during output of a voltage to an X-ray tube). In some embodiments, the control device may obtain the target voltage difference before the bipolar high-voltage generator (e.g., the bipolar high-voltage generator 100) is worked (e.g., at a factory phase, before startup, etc.).

More details regarding the target voltage difference may be found in the description in FIG. 6, which is not repeated here.

In 1420, a working frequency of a first transformer or a second transformer may be adjusted by an inverter circuit based on the target voltage difference.

In some embodiments, to achieve the purpose of balancing an output voltage (e.g., the target voltage difference is zero) or outputting the target voltage difference less than zero, the control device may adjust a working frequency of a first transformer or a second transformer by an inverter circuit, to increase a gain of a cathode voltage output from the bipolar high-voltage generator, and to decrease a gain of an anode voltage output from the bipolar high-voltage generator, thereby making a difference between an absolute value of a target anode voltage and an absolute value of a target cathode voltage satisfies the target voltage difference.

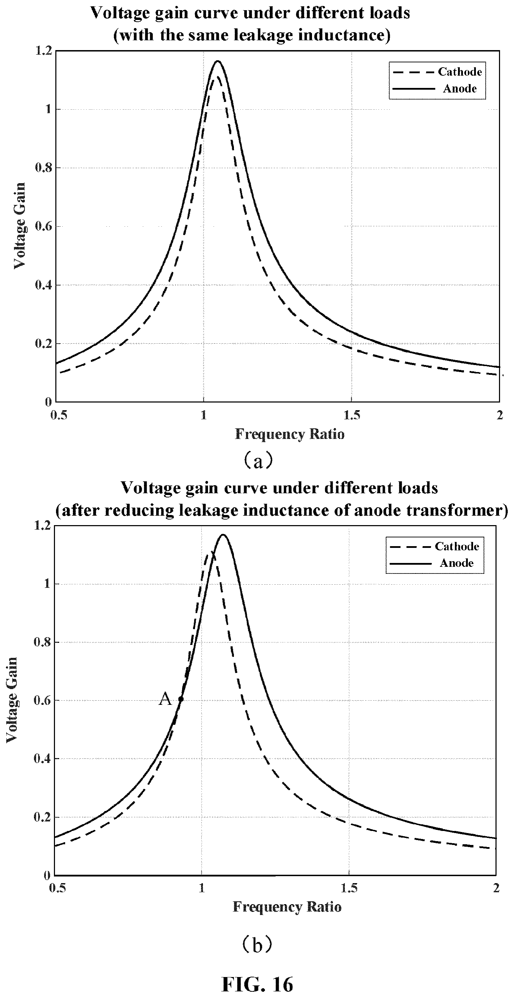

FIG. 15 is a schematic diagram illustrating an equivalent circuit of a bipolar high-voltage generator according to some embodiments of the present disclosure. FIG. 16 is a graph illustrating an output voltage gain curve of a bipolar high-voltage generator according to some embodiments of the present disclosure. The bipolar high-voltage generator 100 shown in FIG. 1 may be equated to a circuit diagram shown in FIG. 15, and based on the equivalent circuit shown in FIG. 15, an output voltage curve graph of the transformer, as shown in FIG. 16, may be obtained. A horizontal coordinate in FIG. 16 is a frequency ratio between the working frequency of the bipolar high-voltage generator and a resonance frequency of the transformer (e.g., a resonance frequency of the first transformer, or a resonance frequency of the second transformer), a vertical coordinate is the gain of the output voltage, a dashed line represents a gain curve of the cathode voltage, and a solid line represents a gain curve of the anode voltage. The resonance frequency of the first transformer is the same as the resonance frequency of the second transformer.

As described above, due to the presence of scattered electrons, a cathode current and an anode current of the bipolar X-ray tube are not equal, and the anode load in the bipolar high-voltage generator is smaller than the cathode load. When circuit parameters of the output circuit of the cathode voltage and the output circuit of the anode voltage in the bipolar high-voltage generator are identical, since the anode load is smaller than the cathode load, the gain of the anode output voltage is always higher than the gain of the cathode output voltage, as shown in the gain curve in FIG. 16(a), which results in an imbalance between the cathode and the anode. However, in practical applications, due to objective factors such as production deviations, leakage inductance parameters of the output circuits of the first transformer and the second transformer in the bipolar high-voltage generator may be different. Even after adjusting the first transformer and the second transformer using the aforementioned manners, the leakage inductance parameters may still be different. As shown in the gain curve in FIG. 16(b), the gain curves of the anode voltage and the cathode voltage intersect at a point A on the left of the resonant peak (the highest point of the curve). By controlling the working frequency of the bipolar high-voltage generator to match a target working frequency corresponding to the point A, the cathode voltage and the anode voltage may be balanced. In some embodiments, as the degree of inconsistency in inductance parameters of the anode output circuit and the cathode output circuit and the difference in power between the anode load and the cathode load vary, the position of the point A may change, meaning that the corresponding target working frequency may also change. Therefore, by setting a target voltage difference as a control objective, i.e., setting the degree of imbalance between the cathode voltage and the anode voltage as the control objective, adjusting the working frequency of the bipolar high-voltage generator, such as by adjusting the working frequency of the first transformer and the working frequency of the second transformer through the inverter circuit, makes the difference between the absolute value of the anode voltage and the absolute value of the cathode voltage fall within the preset range, which achieves the goal of balancing the output voltage.

In some embodiments, when the target voltage difference to be output is greater than zero, i.e., the absolute value of the target anode high-voltage of the final output is greater than the absolute value of the target cathode high-voltage, the control device may, by the inverter circuit, adjust the working frequency of the first transformer or the second transformer, ensuring that the gain of the cathode voltage output by the bipolar high-voltage generator is reduced and the gain of the anode voltage is increased.

In some embodiments, the control device may adjust the working frequency of the first transformer or the second transformer to a target working frequency by the inverter circuit, ensuring that the difference between the absolute value of the target anode voltage output by the bipolar high-voltage generator and the absolute value of the target cathode voltage output by the bipolar high-voltage generator satisfies the target voltage difference. In some embodiments, the target working frequency may be determined by a preset lookup table, real-time computation, a parameter determination model, or the like. For example, the preset lookup table may store the target operating frequencies of at least one of the first transformer or the second transformer corresponding to different target voltage differences; or may store at least one of the target working frequencies of the first transformer corresponding to different target anode voltages, or the target working frequencies of the second transformer corresponding to different target cathode voltages. The preset lookup table may be obtained by statistically analyzing historical data, or by simulating the circuit. As another example, the control device may input the target voltage difference and a circuit parameter and a circuit structure of the bipolar high-voltage generator into the parameter determination model, and determine the target working frequency of at least one of the first transformer or the second transformer based on the output of the parameter determination model.

In some embodiments, after adjusting the working frequency of the first transformer or the second transformer, the control device may obtain a current anode voltage and a current cathode voltage of the bipolar high-voltage generator, determine whether a difference between an absolute value of the current anode voltage and an absolute value of the current cathode voltage falls within the preset range, or reach the target voltage difference. In response to determining that the target voltage difference is not reached or is not within the preset range, the control device continues to adjust the working frequency of the first transformer or the second transformer until the target voltage difference is satisfied. Alternatively, in response to determining that the difference falls outside the preset range, the control device updates the target voltage difference based on the current anode voltage, the current cathode voltage, and the preset range, and further adjusts the working frequency of the first transformer or the second transformer based on the updated target voltage difference until the difference falls within the preset range.

By adjusting the working frequency of the first transformer or the second transformer, the difference between the absolute value of the anode voltage and the absolute value of the cathode voltage output by the bipolar high-voltage generator can be maintained within the preset range. The operation not only enables real-time adjustment during the working of the bipolar high-voltage generator, improving system reliability, but also eliminates the need for additional components in the bipolar high-voltage generator, thereby reducing costs and minimizing the system size.

Embodiments of the present disclosure provide a non-transitory computer-readable storage medium storing computer instructions, and when a computer reads the computer instructions in the storage medium, the computer executes a method, including: obtaining a target voltage difference of a bipolar high-voltage generator; adjusting a working state of at least one of a first transformer and a second transformer based on the target voltage difference, to make a difference between an absolute value of an anode voltage and an absolute value of a cathode voltage output from the bipolar high-voltage generator fall within a preset range. More details regarding the method may be found in FIG. 6 and its related descriptions, which will not be repeated here.

Embodiments of the present disclosure provide a non-transitory computer-readable storage medium storing computer instructions, and when a computer reads the computer instructions in the storage medium, the computer executes a method, including: obtaining a target voltage difference of a bipolar high-voltage generator; adjusting a working frequency of a first transformer or a second transformer by an inverter circuit based on the target voltage difference, to make a difference between an absolute value of an anode voltage and an absolute value of a cathode voltage output by the bipolar high-voltage generator fall within a preset range. More details regarding the method may be found in FIG. 14 and its related descriptions, which will not be repeated here.

The basic concepts have been described above, and it is apparent to a person skilled in the art that the above detailed disclosure is intended only as an example and does not constitute a limitation of the present disclosure. While not expressly stated herein, various modifications, improvements, and amendments may be made to the present disclosure by those skilled in the art. Those types of modifications, improvements, and amendments are suggested in the present disclosure, so those types of modifications, improvements, and amendments remain within the spirit and scope of the exemplary embodiments of the present disclosure.

Also, the present disclosure uses specific words to describe the embodiments of the present disclosure. Such as "an embodiment", "one embodiment", and/or "some embodiment" means a feature, structure, or characteristic associated with at least one embodiment of the present disclosure. Accordingly, it should be emphasized and noted that "an embodiment" or "one embodiment" or "an alternative embodiment" in different places in the present disclosure do not necessarily refer to the same embodiment. In addition, certain features, structures, or characteristics in one or more embodiments of the present disclosure may be suitably combined.

In addition, the order of processing elements and sequences, the use of numerical letters, or the use of other names described herein are not intended to qualify the order of the processes and methods of the present disclosure, unless expressly stated in the claims. While some embodiments of the invention that are currently considered useful are discussed in the foregoing disclosure by way of various examples, it is to be understood that such details serve only illustrative purposes and that additional claims are not limited to the disclosed embodiments, rather, the claims are intended to cover all amendments and equivalent combinations that are consistent with the substance and scope of the embodiments of the present disclosure. For example, although the implementation of various components described above may be embodied in a hardware device, it may also be implemented as a software only solution, e.g., an installation on an existing server or mobile device.

Similarly, it should be noted that in order to simplify the presentation of the disclosure of the present disclosure, and thereby aid in the understanding of one or more embodiments of the invention, the foregoing descriptions of embodiments of the present disclosure sometimes combine a variety of features into a single embodiment, accompanying drawings, or the description thereof. description thereof. However, this method of disclosure does not imply that more features are required for the objects of the present disclosure than are mentioned in the claims. Rather, claimed subject matter may lie in less than all features of a single foregoing disclosed embodiment.

Numbers describing the number of compositions, attributes are used in some embodiments, and it should be understood that such numbers used for the description of embodiments, in some examples, use the modifiers "about ", "approximately", or "generally". Unless otherwise noted, the terms "about," "approximately," or "approximately" indicates that a ±20% variation in the stated number is allowed. Correspondingly, in some embodiments, the numerical parameters used in the specification and claims are approximations, which can change depending on the desired characteristics of individual embodiments. In some embodiments, the numerical parameters should take into account the specified number of valid digits and employ general place-keeping. While the numerical domains and parameters used to confirm the breadth of their ranges in some embodiments of the present disclosure are approximations, in specific embodiments such values are set to be as precise as possible within a feasible range.

For each of the patents, patent applications, patent application disclosures, and other materials cited in the present disclosure, such as articles, books, specification sheets, publications, documents, or the like, the entire contents of which are hereby incorporated herein by reference. Application history documents that are inconsistent with or conflict with the contents of the present disclosure are excluded, as are documents (currently or hereafter appended to the present disclosure) that limit the broadest scope of the claims of the present disclosure. It should be noted that in the event of any inconsistency or conflict between the descriptions, definitions, and/or use of terms in the materials appended to the present disclosure and those set forth herein, the descriptions, definitions and/or use of terms in the present disclosure shall control. use shall prevail.

Finally, it should be understood that the embodiments described in the present disclosure are used only to illustrate the principles of the embodiments of the present disclosure. Other deformations may also fall within the scope of the present disclosure. As such, alternative configurations of embodiments of the present disclosure may be viewed as consistent with the teachings of the present disclosure as an example, not as a limitation. Correspondingly, the embodiments of the present disclosure are not limited to the embodiments expressly presented and described herein.

## Claims

1. A method of controlling a bipolar high-voltage generator, the bipolar high-voltage generator including a first transformer and a second transformer, the bipolar high-voltage generator being connected to a load for supplying a cathode voltage and an anode voltage to the load, the first transformer being provided in an output circuit of the anode voltage, and the second transformer being provided in an output circuit of the cathode voltage, the method comprising:
obtaining a target voltage difference of the bipolar high-voltage generator, wherein the target voltage difference is within a preset range; and
adjusting a working state of at least one of the first transformer and the second transformer based on the target voltage difference, wherein the target voltage difference is defined as a difference between an absolute value of a target anode voltage and an absolute value of a target cathode voltage output from the bipolar high-voltage generator.

2. The method of claim 1, wherein the adjusting a working state of at least one of the first transformer and the second transformer based on the target voltage difference includes:
determining a reference leakage inductance difference between the first transformer and the second transformer based on the target voltage difference; and
determining an adjustment parameter based on the reference leakage inductance difference, the adjustment parameter including at least one of a first adjustment parameter of the first transformer and a second adjustment parameter of the second transformer; and
adjusting a leakage inductance of at least one of the first transformer and the second transformer based on the adjustment parameter.

3. The method of claim 2, wherein the first adjustment parameter includes at least one of a first overlap value between a primary winding and a secondary winding of the first transformer, a winding thickness of the primary winding of the first transformer, and a winding thickness of the secondary winding of the first transformer;
the second adjustment parameter includes at least one of a second overlap value between a primary winding and a secondary winding of the second transformer, a winding thickness of the primary winding of the second transformer, and a winding thickness of the secondary winding of the second transformer.

4. The method of claim 3, wherein the first overlap value corresponds to an overlap dimension of the primary winding and the secondary winding of the first transformer along an axial direction of a first magnetic pillar, and the second overlap value corresponds to an overlap dimension of the primary winding and the secondary winding of the second transformer along an axial direction of a second magnetic pillar.

5. The method of claim 3 or 4, wherein
the bipolar high-voltage generator includes a magnetic core, a first primary winding, a first secondary winding, and a second secondary winding, wherein the magnetic core, the first primary winding, and the first secondary winding form the first transformer, and the magnetic core, the first primary winding, and the second secondary winding form the second transformer;
the first adjustment parameter includes at least one of the first overlap value between the first secondary winding and the first primary winding, and the winding thickness of the first secondary winding; and
the second adjustment parameter includes at least one of the second overlap value between the second secondary winding and the first primary winding, and the winding thickness of the second secondary winding.

6. The method of claim 5, wherein the first primary winding is wound on a magnetic pillar of the magnetic core, the first secondary winding and the second secondary winding are wound side by side on the first primary winding, and the first secondary winding and the second secondary winding are spaced apart along an axial direction of the magnetic pillar; or
the first primary winding is wound on a magnetic pillar of the magnetic core, the second secondary winding is wound on the first primary winding, and the first secondary winding is wound on the second secondary winding.

7. The method of claim 3 or 4, wherein the bipolar high-voltage generator includes a magnetic core, a first primary winding, a second primary winding, a first secondary winding, and a second secondary winding, wherein the magnetic core, the first primary winding, and the first secondary winding form the first transformer, and the magnetic core, the second primary winding, and the second secondary winding form the second transformer;
the first primary winding is wound on a first magnetic pillar of the magnetic core, the first secondary winding is wound on the first primary winding, the second primary winding is wound on a second magnetic pillar of the magnetic core, and the second secondary winding is wound on the second primary winding, the first magnetic pillar being provided opposite to the second magnetic pillar;
the first adjustment parameter includes at least one of: the first overlap value between the first secondary winding and the first primary winding along an axial direction of the first magnetic pillar, and the winding thickness of the first secondary winding;
and the second adjustment parameter includes at least one of: the second overlap value between the second secondary winding and the second primary winding along an axial direction of the second magnetic pillar, and the winding thickness of the second secondary winding.

8. The method of any one of claims 5-7, wherein the adjusting a leakage inductance of at least one of the first transformer and the second transformer based on the adjustment parameter includes:
performing at least one of following adjustment operations to make the bipolar high-voltage generator to produce a target leakage inductance difference, the target leakage inductance difference corresponding to the target voltage difference:
adjusting the first overlap value based on the first adjustment parameter,
adjusting the second overlap value based on the second adjustment parameter,
adjusting the winding thickness of the first secondary winding based on the first adjustment parameter, and
adjusting the winding thickness of the second secondary winding based on the second adjustment parameter.

9. The method of claim 8, wherein the adjusting a leakage inductance of at least one of the first transformer and the second transformer based on the adjustment parameter includes:
performing at least one of following operations to make an overlap difference between the first overlap value and the second overlap value corresponds to the target leakage inductance difference: adjusting the first overlap value based on the first adjustment parameter, and adjusting the second overlap value based on the second adjustment parameter.

10. The method of claim 8 or 9, wherein the adjusting a leakage inductance of at least one of the first transformer and the second transformer based on the adjustment parameter includes:
performing at least one of following operations to make a thickness difference between the winding thickness of the first secondary winding and the winding thickness of the second secondary winding to correspond to the target leakage inductance difference, and the thickness difference is not zero: adjusting the winding thickness of the first secondary winding based on the first adjustment parameter, and adjusting the winding thickness of the second secondary winding based on the second adjustment parameter.

11. The method of any one of claims 2-10, wherein the bipolar high-voltage generator further includes an inverter circuit, the inverter circuit being coupled to an input of the first transformer and an input of the second transformer, and the adjusting a working state of at least one of the first transformer and the second transformer based on the target voltage difference includes:
adjusting, via the inverter, a working frequency of at least one of the first transformer and the second transformer based on the target voltage difference.

12. An X-ray system, comprising:
an X-ray tube, the X-ray tube including a cathode and an anode;
a bipolar high-voltage generator, the bipolar high-voltage generator being connected to the cathode and the anode of the X-ray tube, and the bipolar high-voltage generator supplying a cathode voltage to the cathode of the X-ray tube and an anode voltage to the anode of the X-ray tube, the bipolar high-voltage generator including an inverter circuit, a first transformer, and a second transformer, the first transformer being provided in an output circuit of the anode voltage, and the second transformer being provided in an output circuit of the cathode voltage, the inverter circuit being coupled to an input of the first transformer and an input of the second transformer;
a difference between an absolute value of the anode voltage and an absolute value of the cathode voltage output from the bipolar high-voltage generator falls within a preset range.

13. The X-ray system of claim 12, wherein a working state of at least one of the first transformer and the second transformer is adjustable, and the working state includes at least one of a leakage inductance of the first transformer, a leakage inductance of the second transformer, a working frequency of the first transformer, or a working frequency of the second transformer.

14. The X-ray system of claim 12 or 13, wherein the first transformer or the second transformer includes a primary winding and a secondary winding, at least one of a first overlap value between the primary winding and the secondary winding, a winding thickness of the primary winding, and a winding thickness of the secondary winding is adjustable.

15. The X-ray system of any one of claims 12-14, further comprising an adjustment device, wherein the adjustment device is connected to the bipolar high-voltage generator, and the adjustment device is configured to:
obtain a target voltage difference of the bipolar high-voltage generator, wherein the target voltage difference is within the preset range, and the target voltage difference being defined as a difference between an absolute value of a target anode voltage and an absolute value of a target cathode voltage output from the bipolar high-voltage generator;
determine an adjustment parameter based on the target voltage difference; and
adjust a leakage inductance of at least one of the first transformer and the second transformer based on the adjustment parameter to make the bipolar high-voltage generator to produce a target leakage inductance difference, the target leakage inductance difference corresponding to the target voltage difference.
